# EUROPEAN PATENT SPECIFICATION

(11) **EP 3 360 891 B1**
(45) Date of publication and mention of the grant of the patent: **30.03.2022**
(21) Application number: 18156770.2
(22) Date of filing: 14.02.2018
(51) Int. Cl.: A61K 38/00, C07K 14/47

(54) **CELL PENETRATING PEPTIDES OF HUMAN ORIGIN**
ZELLENPENETRIERENDE PEPTIDE MENSCHLICHEN URSPRUNGS
PEPTIDES DE PÉNÉTRATION CELLULAIRE D'ORIGINE HUMAINE

(30) Priority: 14.02.2017 EP 17156097
(43) Date of publication of application: 15.08.2018
(73) Proprietor: Karlsruher Institut für Technologie, 76131 Karlsruhe (DE)
(72) Inventor: WADHWANI, Parvesh, 76149 Karlsruhe (DE); MÜHLHÄUSER, Philipp, 75443 Ötisheim (DE); REICHERT, Johannes, 77654 Offenburg (DE); CAILLON, Lucie, 41270 La Chapelle Vicomtesse (FR); ULRICH, Anne S., 76229 Karlsruhe (DE); MAISCH, Jan, 76275 Ettlingen (DE); NICK, Peter, 79104 Freiburg (DE); LEE-THEDIECK, Cornelia, 76297 Stutensee (DE); KRAUS, Saskia, 69190 Walldorf (DE); BROCK, Roland, 6525 GA Nijmegen (NL); SCHMIDT, Samuel, 6525 GA Nijmegen (NL); JAIN, Aastha, Delhi-110085 (IN)
(74) Representative: Gille Hrabal Partnerschaftsgesellschaft mbB Patentanwälte

(56) References cited:
- EP-A1- 2 394 665
- US-A1- 2008 020 976
- US-A1- 2009 124 546
- US-A1- 2011 207 657
- US-A1- 2015 239 938
- DANA MARIA COPOLOVICI ET AL: "Cell-Penetrating Peptides: Design, Synthesis, and Applications", ACS NANO, vol. 8, no. 3, 25 March 2014 (2014-03-25), pages 1972-1994, XP055341873, US ISSN: 1936-0851, DOI: 10.1021/nn4057269
- MAREN PAULMANN ET AL: "Structure-Activity Analysis of the Dermcidin-derived Peptide DCD-1L, an Anionic Antimicrobial Peptide Present in Human Sweat", JOURNAL OF BIOLOGICAL CHEMISTRY, vol. 287, no. 11, 9 March 2012 (2012-03-09), pages 8434-8443, XP055395986, US ISSN: 0021-9258, DOI: 10.1074/jbc.M111.332270
- H. STEFFEN ET AL: "Naturally Processed Dermcidin-Derived Peptides Do Not Permeabilize Bacterial Membranes and Kill Microorganisms Irrespective of Their Charge", ANTIMICROBIAL AGENTS AND CHEMOTHERAPY, vol. 50, no. 8, 1 August 2006 (2006-08-01) , pages 2608-2620, XP055108670, ISSN: 0066-4804, DOI: 10.1128/AAC.00181-06
- IWASE YUKO ET AL: "Use of a non-covalent cell-penetrating peptide strategy to enhance the nasal delivery of interferon beta and its PEGylated form", INTERNATIONAL JOURNAL OF PHARMACEUTICS, ELSEVIER, AMSTERDAM, NL, vol. 510, no. 1, 22 June 2016 (2016-06-22) , pages 304-310, XP029659372, ISSN: 0378-5173, DOI: 10.1016/J.IJPHARM.2016.06.054
- SIEGBERT RIEG ET AL: "Generation of Multiple Stable Dermcidin-Derived Antimicrobial Peptides in Sweat of Different Body Sites", JOURNAL OF INVESTIGATIVE DERMATOLOGY, vol. 126, no. 2, 22 December 2005 (2005-12-22), pages 354-365, XP055112395, ISSN: 0022-202X, DOI: 10.1038/sj.jid.5700041
- MARC BURIAN ET AL: "The secrets of dermcidin action", INTERNATIONAL JOURNAL OF MEDICAL MICROBIOLOGY, vol. 305, no. 2, 1 February 2015 (2015-02-01), pages 283-286, XP055465960, DE ISSN: 1438-4221, DOI: 10.1016/j.ijmm.2014.12.012

## Description

### FIELD OF THE INVENTION

The present invention relates to novel cell-penetrating peptides as a carrier for the transport of cargo molecules into cells. The novel cell-penetrating peptides of the present invention have been found to be very efficient and, since they are derived from a human amino acid sequence, are neither immunogenic nor toxic.

### BACKGROUND OF THE INVENTION AND PRIOR ART

Administration of macromolecules into the interior of cells across the membrane bilayer is usually not possible. The plasma membrane of the cells constitutes a hydrophobic and strictly controlled barrier, which prevents the uncontrolled penetration of bioactive molecules. However, several treatments (medical and/or cosmetic) face the problem to introduce polar molecules, peptides, proteins, nucleic acid derivatives, polymers, nanoparticles or other pharmaceutically or cosmetically active ingredients in a targeted manner into cells, in particular if such molecules or active compounds are not of endogenous origin and thus do not enter the cells via a natural transport machinery. Various approaches based on biological, chemical and physical methods exist to achieve an appropriate translocation of the respective molecules or active compounds through the hydrophobic and thus impermeable lipid layer of the cell membranes.

In recent years, cell-penetrating peptides (cell-penetrating peptide: CPP) have been discussed as a promising approach for introducing various kinds of bioactive molecules for therapeutic or diagnostic purposes into cells. Such CPPs are short peptide sequences which are able to carry even large macromolecules with molecular weights that are several times higher than their own, for example proteins, to an efficient extent through biological membranes. An ideal CPP translocates independent of membrane-bound transport complexes or specific receptors or energy dependent pathways.

The first CPPs (Penetratin and Tat) discovered more than 20 years ago were exclusively derived from the natural occurring protein sequences of Antennapedia Homeodomain and HIV [D. Derossi, A.H. Joliot, G. Chassaing, A. Prochiantz, The 3rd Helix of the Antennapedia Homeodomain Translocates through Biological-Membranes, J Biol Chem, 269 (1994) 10444-10450*;* and E. Vivés, P. Brodin, B. Lebieu, A truncated HIV-1 Tat protein basic domain rapidly translocates through the plasma membrane and accumulates in the cell nucleus, J Biol Chem 269 (1997) 16010-16017*].* In the subsequent period, a large number of further CPPs have been described, both on basis of natural sequences, preferably partial sequences of proteins, as well as synthetic model sequences, e.g. MAP or polyarginine (R8 or R9). A general overview about the development of CPPs is given in the following publications from the Langel-group [D.M. Copolovici, K. Langel, E. Eriste, U. Langel, Cell-Penetrating Peptides: Design, Synthesis, and Applications, ACS Nano, 8 (2014) 1972-1994*;* M. Mäe, Ü. Langel, Cell-penetrating peptides as vectors for peptide, protein and oligonucleotide delivery, Current Opinion in Pharmacology, 6 (2006) 509-514; M. Lindgren, M. Hällbrink, A. Prochiantz, Ü. Langel, Cell-penetrating peptides, Trends in Pharmacological Sciences, 21 (2000) 99-103*].*

Usually, the protein-derived CPPs consist of that minimal amino acid sequence of a protein which allows the protein to translocate the membrane either independently or in presence of a cargo. In contrast, the model CPPs are usually designed to form amphipathic α-helices and/or to mimic the secondary structure of known CPPs. For various CPPs it has been shown that they translocate the plasma membrane of eukaryotic cells via an apparently energy-independent path, which provides in general the possibility to use such cell-penetrating peptides as transport sequences (transport vectors, carriers, Trojans) for pharmacologically interesting substances, such as peptides, proteins, oligonucleotides or antisense RNA molecules.

Furthermore, CPPs have also been described in patent applications, such as in WO 2003/106491, US 2007/0129305, EP2394665 and WO 2014/041505 or its equivalent US 2015/0239938.

The development of CPPs is promoted worldwide with the aim of developing efficient and non-toxic transporters or "delivery systems" to be able to introduce pharmaceutically relevant substances into cells, which, *per se,* are not able to penetrate the cell membrane. To date, almost 1000 peptide sequences have been described, and a few of them have already reached clinical trials. But a general problem here is that although the already known CPP sequences act very effectively, they also have undesired side-effects, such as membrane damage, disturbances of the function of membrane proteins or other toxic effects as well as immunogenic reactions. As a consequence, their use always requires a thorough benefit-risk-analysis.

It is therefore, needed that improved CPPs are developed which are able to carry a wide range of bioactive molecules into cells with high efficiency, low toxicity, without activating the immune system and in a targeted manner.

Further, an example of non-covalent mode of cargo attachment has been reported in literature with well-known CPPs like Tat and Penetratin [Yuko Iwase, Noriyasu Kamei, EI-Sayed Khafagy, Mitsuko Miyamoto, Use of a non-covalent cell-penetrating peptide strategy to enhance the nasal delivery of interferon beta and its PEGylated form, International Journal of Pharmaceutics 510 (2016) 304-310]*.*

In the beginning of 2000 the so-called dermcidin-peptides have been discovered in the human sweat. Dermcidin (DCD, 110 amino acids) is expressed in human eccrine perspiratory glands (sweat glands), secreted into the sweat and transported to the skin surface, where its proteolytic cleavage takes place [B. Schittek, R. Hipfel, B. Sauer, J. Bauer, H. Kalbacher, S. Stevanovic, M. Schirie, K. Schroeder, N. Blin, F. Meier, G. Rassner, C. Garbe, Dermcidin: a novel human antibiotic peptide secreted by sweat glands, Nat immunol, 2 (2001) 1133-1137*;* S. Rieg, S. Seeber, H. Steffen, A. Humeny, H. Kalbacher, S. Stevanovic, A. Kimura, C. Garbe, B. Schittek, Generation of multiple stable dermcidin-derived antimicrobial peptides in sweat of different body sites, J invest Dermatol, 126 (2006) 354-365]*.*

Dermcidin-peptides have been described to have antimicrobial activity such as in particular the C-terminal fragment DCD-1L (48 amino acids) as well as the N-terminal cleavage product thereof SSL-25 (25 amino acids) [H. Steffen, S. Rieg, I. Wiedemann, H. Kalbacher, A. Deeg, H.G. Sahi, A. Peschei, F. Gotz, C. Garbe, B. Schittek, Naturally processed dermcidin-derived peptides do not permeabilize bacterial membranes and kill microorganisms irrespective of their charge, Antimicrob Agents Chemotherapy, 50 (2006) 2608-2620*;* M. Paulmann, T. Arnold, D. Linke, S. Ozdirekcan, A. Kopp, T. Gutsmann, H. Kalbacher, I. Wanke, V. J. Schuenemann, M. Habeck, J. Bürck, A. S. Ulrich, B. Schittek, Structure-activity analysis of the dermcidin-derived peptide DCD-1L, an anionic antimicrobial peptide present in human sweat, Journal of Biological Chemistry, 287, No. 11 (2012) 8434-8443]. With their significant amphiphilic helical structure they are very similar to many known antimicrobial peptides.

For example, WO 2002/100895 and US 2008/0020976 relate to dermcidin (DCD) and its suitability as an antimicrobial agent for treating skin diseases.

Dermcidin-derived peptides with antimicrobial activity and their pharmaceutical use for treating inflammatory conditions and microbial skin infections have been described in WO 2007/133730 and in WO 2011/0361174.

Due to their antimicrobial activity, also cosmetic applications of dermcidin-derived peptides have been described. For example, WO 2014/207181 describes the use of DCD-1 or DCD-2 as a modulator of the bacterial skin flora, which results in the treatment of body odor, i.e. the effect of deodorization.

Further cosmetic applications of dermcidin-derived peptides comprise the use of DCD-1L and DCD-1 as a cosmetic biomarker for mature and dry skin as described in WO 2012/038929 and its corresponding equivalent US 2013/0324476 or as a cosmetic biomarker for oily skin and for topically treating acne by inhibiting the growth of *Propionibacterium acnes* as described in WO 2014/199106.

Besides the cosmetic biomarkers it has also been described in US 2011/0195133 to use a further dermcidin-derived peptide E-R11 as a biomarker for lung cancer.

Furthermore, other peptides such as phylloseptin and dermaseptin as described in WO 2011/073440 with antimicrobial activity and deodorization properties can be mentioned.

### OBJECT TO BE SOLVED

The object to be solved was to provide novel CPPs which do not have the disadvantages of the prior art CPPs. In particular, the novel CPPs should be able to carry a wide range of bioactive molecules into cells, should act with high efficiency, show low or even no toxicity and/or should act without activating the immune system. Further, novel CPPs should be provided for any application by easy and cost effective production methods.

The inventors of the present invention have surprisingly found that a group of peptides derived from dermcidin, hereinafter also designated as the so-called dermcidines, can be used as cell-penetrating peptides.

The inventors have shown, that the dermcidines according to the present invention are able to penetrate very efficiently into cells. Due to their human origin they are neither immunogenic nor toxic and as has been shown by the present inventors, they further do not cause significant membrane damages.

These findings are insofar surprising, as the occurrence of dermcidines in human sweat on the skin surface suggests that their biological effect is intended to be localized at said external site, i.e. either on the skin surface or at most in the upper dermal layer. Further, the underlying mechanism of action of amphiphilic peptides with antimicrobial activity is usually the damage of the bacterial membranes as the main target, which often is accompanied by a permeabilization and damage of cell membranes and therefore, could be an undesirable side effect. It has now surprisingly been found by the inventors of the present invention that in contrast to the so far described antimicrobial activity of dermcidines with the underlying mechanism of damaging bacterial membranes that dermcidines according to the present invention are particularly suitable as CPPs without undesired side effects of damaging eukaryotic cell membranes.

The inventors of the present invention have thus surprisingly found that a group of peptides derived from dermcidin, the so-called dermcidines, have cell-penetrating properties and are thus able to carry a wide range of bioactive molecules into cells. The inventors have further surprisingly found that the aforesaid type of side-effect of permeabilization and damage of eukaryotic cell membranes does not occur with the dermcidines of the present invention.

In addition, the inventors of the present invention could, in contrast to the disclosure of the prior art, not detect any antimicrobial activity, neither for DCD-1L nor for SSL-25. This has also been already confirmed by studies of López-García et al. [B. López-García , P. H. A. Lee and R. L. Gallo Expression and potential function of cathelicidin antimicrobial peptides in dermatophytosis and tinea versicolor Journal of Antimicrobial Chemotherapy (2006) 57, 877-882*]*. Further studies on antimicrobial activity have been described by M. Murakami et al. [M. Murakami, T. Ohtake, R.A. Dorschner, B. Schittek, C. Garbe, R.L. Gallo, Cathelicidin anti-microbial peptide expression in sweat, an innate defense system for the skin, J Invest Dermatol, (2002), 119 1090-1095*;* M. Murakami, B. Lopez-Garcia, M. Braff, R.A. Dorschner, R.L. Gallo, Postsecretory processing generates multiple cathelicidins for enhanced topical antimicrobial defense, J Immunol, (2004), 172 3070-3077]*.*

The present invention thus solves the object of the invention by providing novel CPPs, which are derived from peptide sequences of human origin and which are neither toxic nor immunogenic and which do not cause any undesired membrane damage even at high concentration.

### DETAILED DESCRIPTION OF THE INVENTION

The present invention relates to novel cell-penetrating peptides (CPPs) as a carrier for the transport of pharmaceutically, cosmetically or biotechnologically interesting molecules (cargo molecules) into cells.

The novel cell-penetrating peptides (CPPs) are derived from the protein dermcidin, present in human sweat composed of 110 amino acids having the amino acid sequence SEQ ID 88 as shown below:

### Amino Acid Sequence SEQ ID 88 (Dermcidin)

Dermcidin comprises a signal peptide with 19 amino acids and its gene is located on chromosome 12, locus 12q13.1.

Dermcidin *per se* is widely known and has been described in detail for example in the above mentioned prior art (*Schittek et al. 2001 or Rieg et al. 2006*) as well as in the above cited patent applications WO 2002/100895, WO 2007/133730, US 2008/0020976 WO 2011/0361174, WO2012038929A1 (with its corresponding US 2013/0324476), WO 2014/199106 or WO2014207181, the disclosure of which is enclosed in the present invention by reference.

In the context of the present invention the term "dermcidines" comprises dermcidin with the SEQ ID 88 as well as isoforms, fragments or analogues thereof. Examples comprise two isoforms as listed in the UniProt base, a shorter one, isoform 1 (DCD-1), and a longer one, isoform 2 (DCD-2). As mentioned above, due to proteolytic post-translational processing (essentially by cathepsin D) which takes place in the sweat, the protein precursor gives rise to numerous peptides whose length ranges from 25 to 48 amino acids. They may be derived from the C-terminal part of the dermcidin protein as well as from the N-terminal part of the dermcidin protein and are also covered by the term "dermcidines" in the sense of the present invention.

The peptide sequences disclosed herein are shown proceeding from left to right, with the left end of the sequence being the N-terminus of the peptide and the right end of the sequence being the C-terminus. The peptides employed here include naturally occurring proteinogenic L-amino acids and are designated by their conventional single-letter code.

In the present specification naturally occurring proteinogenic amino acids are usually designated by their conventional single-letter abbreviations (e.g. in particular in the sequence listings). Alternatively, they can also be referred to by their three-letter abbreviations or by their full name as shown in Table 1 below:

**Table 1:**

| **3-Letter** | **1-Letter** | **Amino Acid** | | **3-Letter** | **1-Letter** | **Amino Acid** |
|---|---|---|---|---|---|---|
| Ala | A | Alanine | | Leu | L | Leucine |
| Arg | R | Arginine | | Lys | K | Lysine |
| Asn | N | Asparagine | | Met | M | Methionine |
| Asp | D | Aspartic acid | | Phe | F | Phenylalanine |
| Cys | C | Cysteine | | Pro | P | Proline |
| Glu | E | Glutamic acid | | Ser | S | Serine |
| Gln | Q | Glutamine | | Thr | T | Threonine |
| Gly | G | Glycine | | Trp | W | Tryptophan |
| His | H | Histidine | | Tyr | Y | Tyrosine |
| Ile | I | Isoleucine | | Val | V | Valine |

The term "L-amino acid," as used herein, refers to the "L" isomeric form of an amino acid, and conversely the term "D-amino acid" refers to the "D" isomeric form of an amino acid. It is a conventional manner to indicate the L-amino acid with capital letters such as Ala / A, Arg / R, etc. and the D-amino acid with small letters such as ala / a, arg / r, etc..

Examples of C-terminal peptide fragments derived from dermcidin comprise DCD-1L (48 amino acids) and DCD-1 (47 amino acids). Further shorter C-terminal fragments include for example SSL-46 (46 amino acids), SSL-45 (45 amino acids) and SSL-29 (29 amino acids). N-terminal peptide fragments derived from DCD-1L comprise SSL-25 (25 amino acids) and analogues thereof such as for example SSL-25 E5A, SSL-25 D9A, SSL-25 D24A, SSL-25 E5AD9AD24A, as well as SSL-23 (23 amino acids), SSL 21 (21 amino acids), SSL-19 (19 amino acids), SSL-17 (17 amino acids), SSL15 (15 amino acids), SSL-13 (13 amino acids) and analogues thereof such as for example SSL-19 D9A (19 amino acids), SSL-17 D9A (17 amino acids), SSL-15 D9A (15 amino acids), SSL-13 D9A, as well as SSL-12 (12 amino acids), SSL-11 (11 amino acids), SSL-10 (10 amino acids), SSL-9 (9 amino acids), SSL-8 (8 amino acids), SSL-7 (7 amino acids), SSL-6 (6 amino acids), and analogues thereof such as for example SSL-12 D9A, SSL-11 D9A, SSL-11 E5A D9A, SSL-10 D9A, SSL-9 E5A D9K, SSL-9 E5K D9A, SSL-9 E5K D9K, SSL-8 E5K, SSL-7 E5A, SSL-7 E5K, SSL-6 E5A, SSL-6 E5K, as well as in particular all fragments and analogues thereof as shown in Table 2 below.

The dermcidin-derived peptides (DPPs) according to the present invention have the following consecutive sequence of formula (I)

S-S-L-L-X1-K-G-L-X2-X3-X4-X5-X6-X7 (I)

including retro-, inverso- and retroinverso-forms thereof (as defined below), wherein
- X1: is any L- or D-amino acid;
- X2: is any L- or D-amino acid or is absent;
- X3: is any L- or D-amino acid or is absent;
- X4: is absent or an amino acid sequence comprising from 1 to 10 amino acids of the consecutive amino acid sequence A-Y1-Y2-A-V-G-G-L-G-Y3, wherein Y1, Y2 and Y3 are independently any L- or D-amino acid or are absent
- X5: is absent or an amino acid sequence comprising from 1 to 10 amino acids of the consecutive amino acid sequence L-G-Y4-Y5-A-V-E-D-L-E, wherein Y4 and Y5 are independently any L- or D-amino acid or are absent;
- X6: is absent or an amino acid sequence comprising from 1 to 10 amino acids of the consecutive amino acid sequence S-V-G-K-G-A-V-H-D-V;
- X7: is absent or an amino acid sequence comprising from 1 to 8 amino acids of the consecutive amino acid sequence K-D-V-L-D-S-V-L;
wherein in the case that one of X2 to X6 is absent the subsequent amino acids X3 to X7 are also absent.

In a further aspect of the present invention in the consecutive sequence of formula (I) above, defining the dermcidin-derived peptides (DPPs) according to the present invention, X2 to X7 are absent and further one or both of the remaining right terminal amino acids -G-L are also absent, thus defining an amino acid sequence of 7 or 6 amino acids.

Accordingly, in a further aspect of the present invention the dermcidin-derived peptides (DPPs) according to the present invention, including retro-, inverso- and retroinverso-forms thereof (as defined below), and derivatives and analogues derived therefrom (and as defined below), are defined by the following consecutive sequence of formula (II)

**S-S-L-L-X1 -K-Z1** (II)

including retro-, inverso- and retroinverso-forms thereof (as defined below), wherein
- X1: is any L- or D-amino acid; and
- Z1: is G or g or is absent.

The dermcidin-derived peptides (DPPs) according to the present invention represent different fragments of the dermcidin protein having different amino acid chain lengths. Therefore, if in the consecutive sequence of formula (I) one of X2, X3, X4, X5 or X6 is absent this indicates the end of the chain and therefore the subsequent amino acids X3, X4, X5, X6 and/or X7, respectively, are necessarily also absent.

Similarly, if in the consecutive sequence of formula (II) Z1 is absent this indicates the end of the chain.

From the consecutive sequence of formulae (I) and (II) above it becomes apparent that the key fragment of the dermcidin-derived peptides (DPPs) of the present invention comprise at least the following consecutive amino acid sequence (Ia)

**S-S-L-L-X1-K-G-L-X2** (Ia)

wherein X1 and X2 have the meaning as defined above for the consecutive sequence of formula (I); or
at least the above defined consecutive amino acid sequence (II)

   **S-S-L-L-X1-K-Z1** (II)
with the meaning of X1 and Z1 as defined above.

In particular, the initial consecutive amino acid sequence (III)

**S-S-L-L** (III)

can be identified as the key-sequence of the DPPs of the present invention.

In the consecutive sequences of formulae (I), (Ia), (II) and (III) and in the specific sequences as defined anywhere in the present invention, such as e.g. in Table 2 below the amino acids are indicated by using capital letters, referring to the L-amino acid. However, any or all of said amino acids may also be present in its respective D-form.

A further aspect of the present invention relates to the dermcidin-derived peptides (DPPs) according to the consecutive sequence of formula (I) or (Ia) above, wherein
- X1: is any L- or D-amino acid;
- X2: is any L- or D-amino acid or is absent;
- X3: is any L- or D-amino acid or is absent;
- X4: is absent or an amino acid sequence comprising from 1 to 10 amino acids of the amino acid sequence A-Y1-Y2-A-V-G-G-L-G-Y3, wherein Y1, Y2 and Y3 are independently any L- or D-amino acid or are absent;
- X5: is absent or an amino acid sequence comprising from 1 to 5 amino acids of the amino acid sequence L-G-Y4-Y5-A, wherein; Y4 and Y5 are independently any L- or D-amino acid or are absent;
- X6: is absent;
- X7: is absent;
wherein in the case that one of X2 to X4 is absent the subsequent amino acids X3 to X5 are also absent.

A further aspect of the present invention relates to the dermcidin-derived peptides (DPPs) according to the consecutive sequence of formula (I) or (Ia) above, wherein
- X1: is any L- or D-amino acid;
- X2: is any L- or D-amino acid or is absent;
- X3: is any L- or D-amino acid or is absent;
- X4: is absent or an amino acid sequence comprising from 1 to 10 amino acids of the amino acid sequence A-Y1-Y2-A-V-G-G-L-G-Y3, wherein Y1, Y2 and Y3 are independently any L- or D-amino acid or are absent;
- X5: is absent;
- X6: is absent;
- X7: is absent;
wherein in the case that X2 or X3 is absent the subsequent amino acid X3 and/or X4 is also absent.

A further aspect of the present invention relates to the dermcidin-derived peptides (DPPs) according to the consecutive sequence of formula (I) or (Ia) above, wherein
- X1: is any L- or D-amino acid;
- X2: is any L- or D-amino acid or is absent;
- X3: is any L- or D-amino acid or is absent;
- X4: is absent or an amino acid sequence comprising from 1 to 5 amino acids of the amino acid sequence A-Y1-Y2-A-V, wherein Y1 and Y2 are independently any L- or D-amino acid or are absent;
- X5: is absent;
- X6: is absent;
- X7: is absent;
wherein in the case that X2 or X3 is absent the subsequent amino acid X3 and/or X4 is also absent.

A further aspect of the present invention relates to the dermcidin-derived peptides (DPPs) according to the consecutive sequence of formula (I) or (Ia) above, wherein
- X1: is any L- or D-amino acid;
- X2: is any L- or D-amino acid or is absent;
- X3: is any L- or D-amino acid or is absent;
- X4: is absent or an amino acid sequence comprising from 1 to 3 amino acids of the amino acid sequence A-Y1-Y2, wherein Y1 and Y2 are independently any L- or D-amino acid or are absent;
- X5: is absent;
- X6: is absent;
- X7: is absent;
wherein in the case that X2 or X3 is absent the subsequent amino acid X3 and/or X4 is also absent.

A further aspect of the present invention relates to the dermcidin-derived peptides (DPPs) according to the consecutive sequence of formula (I) or (Ia) above, wherein
- X1: is any L- or D-amino acid;
- X2: is any L- or D-amino acid or is absent;
- X3: is any L- or D-amino acid or is absent;
- X4: is A or is absent;
- X5: is absent;
- X6: is absent;
- X7: is absent;
wherein in the case that X2 or X3 is absent the subsequent amino acid X3 and/or X4 is also absent.

Further aspects of the present invention relate to the dermcidin-derived peptides (DPPs) according to the consecutive sequence of formula (I) or (Ia) above as described in any of the aforesaid aspects of the invention, wherein
- X1: is E, A or K, or e, a or k; or
- X1: is E or A, or e or a; or
- X1: is E or K, or e or k; or
- X1: is A or K, or a or k; or
- X1: is E or e; or
- X1: is A or a; or
- X1: is K or k;
and/or
- X2: is D, A or K, or d, a or k or is absent; or
- X2: is D or A or d or a; or
- X2: is D or K, or d or k; or
- X2: is A or K, or a or k; or
- X2: is D or d; or
- X2: is A or a; or
- X2: is K or k; or
- X2: is absent;
and/or
- X3: is G or g or is absent; or
- X3: is G or g;
- X3: is absent;
and/or
Y1, Y2, Y3, Y4 and Y5 are independently selected from the group consisting of E, A, K, D, e, a, k and d or are absent; preferably
- Y1: is K or k or is absent;
and/or
- Y2: is K or k or is absent;
and/or
- Y3: is K or k or is absent;
and/or
- Y4: is K or k or is absent;
and/or
- Y5: is D or d or A or is absent; preferably
- Y5: is D or d or is absent; preferably
- Y5: is absent.

A further aspect of the present invention relates to the dermcidin-derived peptides (DPPs) according to the consecutive sequence of formula (II) above and as described in any of the aspects of the present invention, wherein
- X1: is E, A or K, or e, a or k; or
- X1: is E or A, or e or a; or
- X1: is E or K, or e or k; or
- X1: is A or K, or a or k; or
- X1: is E or e; or
- X1: is A or a; or
- X1: is K or k; and

- Z1: is G or g or is absent.

A further aspect of the present invention relates to the dermcidin-derived peptides (DPPs) according to the consecutive sequence of formula (II) above and as described in any of the aspects of the present invention, wherein
- X1: is E, A or K, or e, a or k; or
- X1: is E or A, or e or a; or
- X1: is E or K, or e or k; or
- X1: is A or K, or a or k; or
- X1: is E or e; or
- X1: is A or a; or
- X1: is K or k; and

- Z1: is G or g.

A further aspect of the present invention relates to the dermcidin-derived peptides (DPPs) according to the consecutive sequence of formula (II) above and as described in any of the aspects of the present invention, wherein
- X1: is E, A or K, or e, a or k; or
- X1: is E or A, or e or a; or
- X1: is E or K, or e or k; or
- X1: is A or K, or a or k; or
- X1: is E or e; or
- X1: is A or a; or
- X1: is K or k; and

- Z1: is absent.

For clarifying reasons it is mentioned that in the case that any one of X4, X5, X6 or X7 refers to "an amino acid sequence comprising from x to y amino acids" said range indicates the length of the respective amino acid sequence from left to right. For example, if X4 is defined to be an amino acid sequence comprising from 1 to 5 amino acids of the amino acid sequence A-Y1-Y2-A-V, then X4 may be a single amino acid "A", a sequence of two amino acids "A-Y1", a sequence of three amino acids "A-Y1-Y2", a sequence of four amino acids "A-Y1-Y2-A" or a sequence of five amino acids "A-Y1-Y2-A-V". Or, if X5 is defined to be an amino acid sequence comprising from 1 to 10 amino acids of the amino acid sequence L-G-Y3-Y4-A-V-E-D-L-E, then X5 may be a single amino acid "L", a sequence of two amino acids "L-G", a sequence of three amino acids "L-G-Y3", a sequence of four amino acids "L-G-Y3-Y4", a sequence of five amino acids "L-G-Y3-Y4-A", a sequence of six amino acids "L-G-Y3-Y4-A-V", a sequence of seven amino acids "L-G-Y3-Y4-A-V-E", a sequence of eight amino acids "L-G-Y3-Y4-A-V-E-D", a sequence of nine amino acids "L-G-Y3-Y4-A-V-E-D-L", or a sequence of ten amino acids "L-G-Y3-Y4-A-V-E-D-L-E"; and so on.

In particular, the dermcidin-derived peptides (DPPs) according to the present invention are selected from the group consisting of DCD-1L, DCD-1, SSL-46, SSL-45, SSL-29, SSL-25, SSL-25 E5A, SSL-25 D9A, SSL-25 D24A, SSL-25 E5AD9AD24A, SSL-24, SSL-24 E5A, SSL-24 D9A, SSL-24 D24A, SSL-24 E5A D9A D24A, SSL-23, SSL-23 E5A, SSL-23 D9A, SSL-23 E5A D9A, SSL-22, SSL-22 E5A, SSL-22 D9A, SSL-22 E5A D9A, SSL-21, SSL-21 E5A, SSL-21 D9A, SSL-21 E5A D9A, SSL-20, SSL-20 E5A, SSL-20 D9A, SSL-20 E5A D9A, SSL-19, SSL-19 E5A, SSL-19 D9A, SSL-19 E5A D9A, SSL-18, SSL-18 E5A, SSL-18 D9A, SSL-18 E5A D9A, SSL-17, SSL-17 E5A, SSL-17 D9A, SSL-17 E5A D9A, SSL-16, SSL-16 E5A, SSL-16 D9A, SSL-16 E5A D9A, SSL-15, SSL-15 E5A, SSL-15 D9A, SSL-15 E5A D9A, SSL-14, SSL-14 E5A, SSL-14 D9A, SSL-14 E5A D9A, SSL-13, SSL-13 E5A, SSL-13 D9A, SSL-13 E5A D9A, SSL-12, SSL-12 E5A, SSL-12 D9A, SSL-12 E5A D9A, SSL-11, SSL-11 E5A, SSL-11 D9A, SSL-11 E5A D9A, SSL-10, SSL-10 E5A, SSL-10 D9A, SSL-10 E5A D9A, SSL-9, SSL-9 E5A, SSL-9 D9A, SSL-9 E5A D9A, SSL-9 E5A D9K, SSL-9 E5K D9A, SSL-9 E5K D9K, SSL-8, SSL-8 E5A, SSL-8 E5K, SSL-7, SSL-7 E5A, SSL-7 E5K, SSL-6, SSL-6 E5A, SSL-6 E5K including retro-, inverso- and retroinverso-forms thereof (as defined below), and derivatives and analogues derived therefrom.

The above mentioned dermcidin-derived peptides are in particular represented by the following amino acid sequences (with the amino acids according to the single-letter code indicating the respective L-isoform):

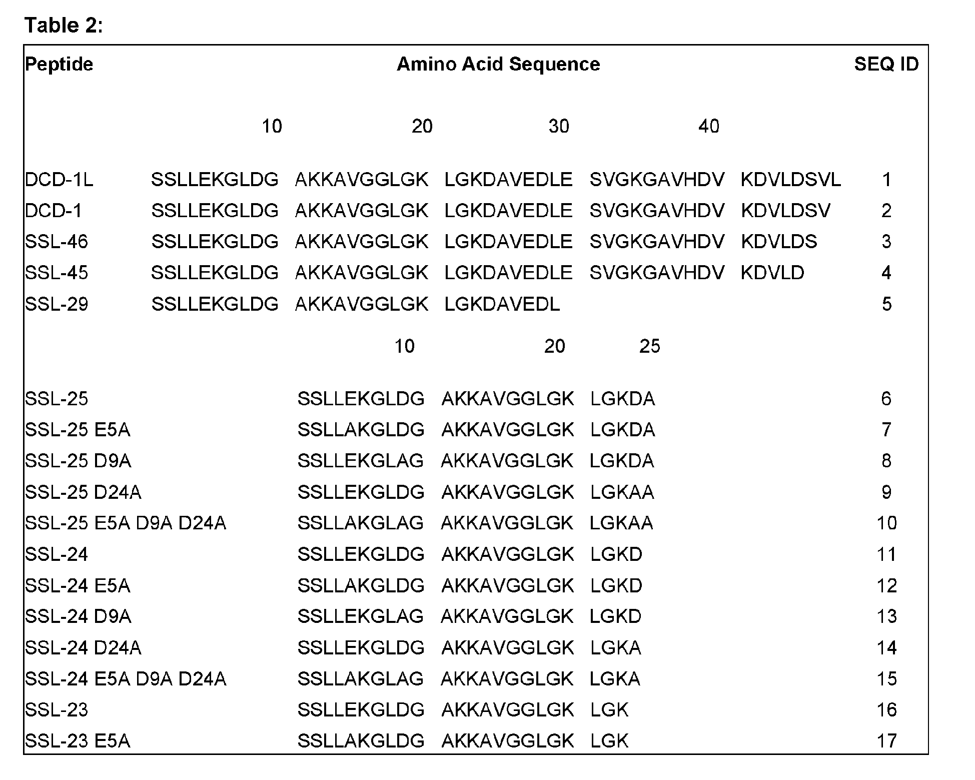

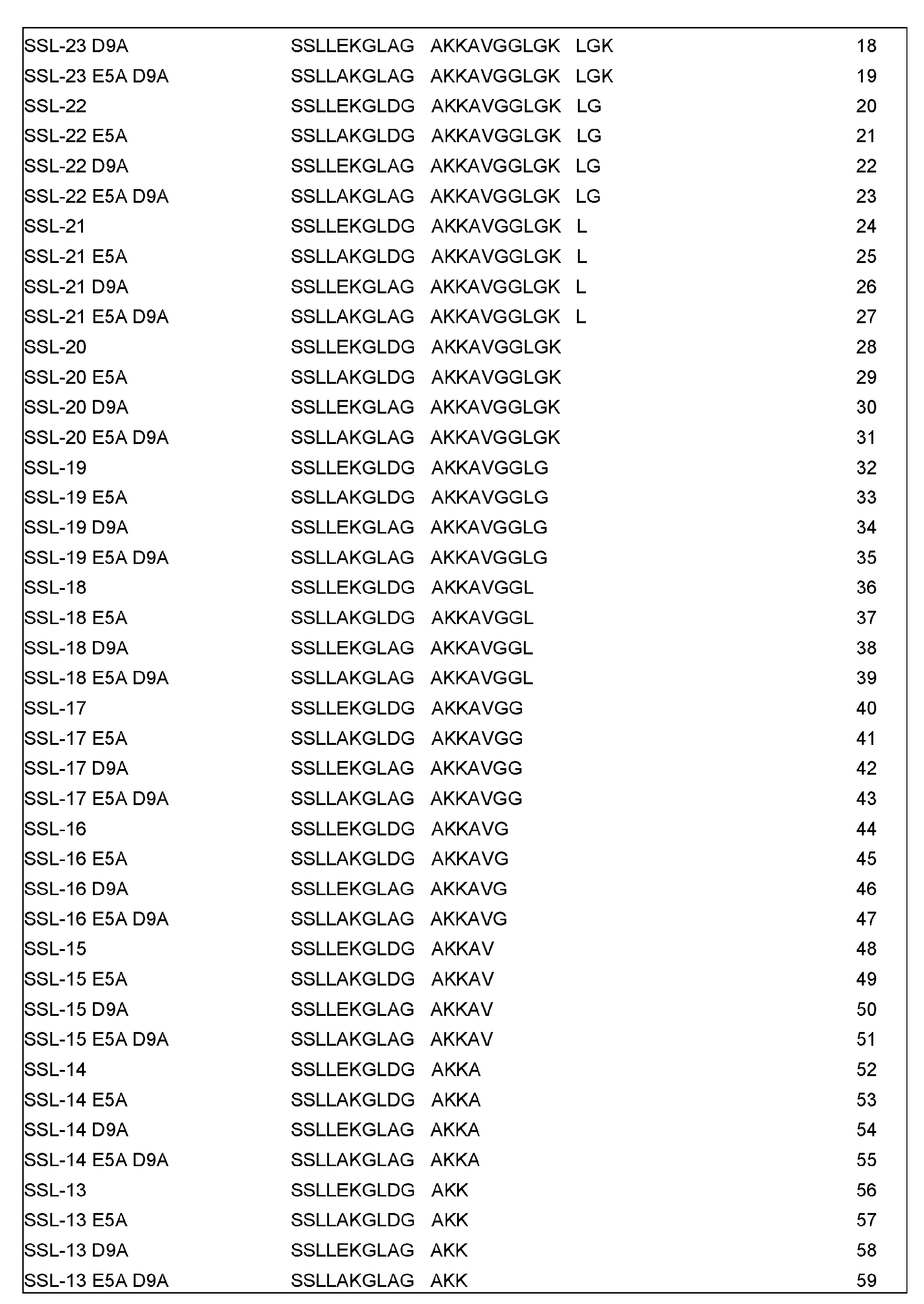

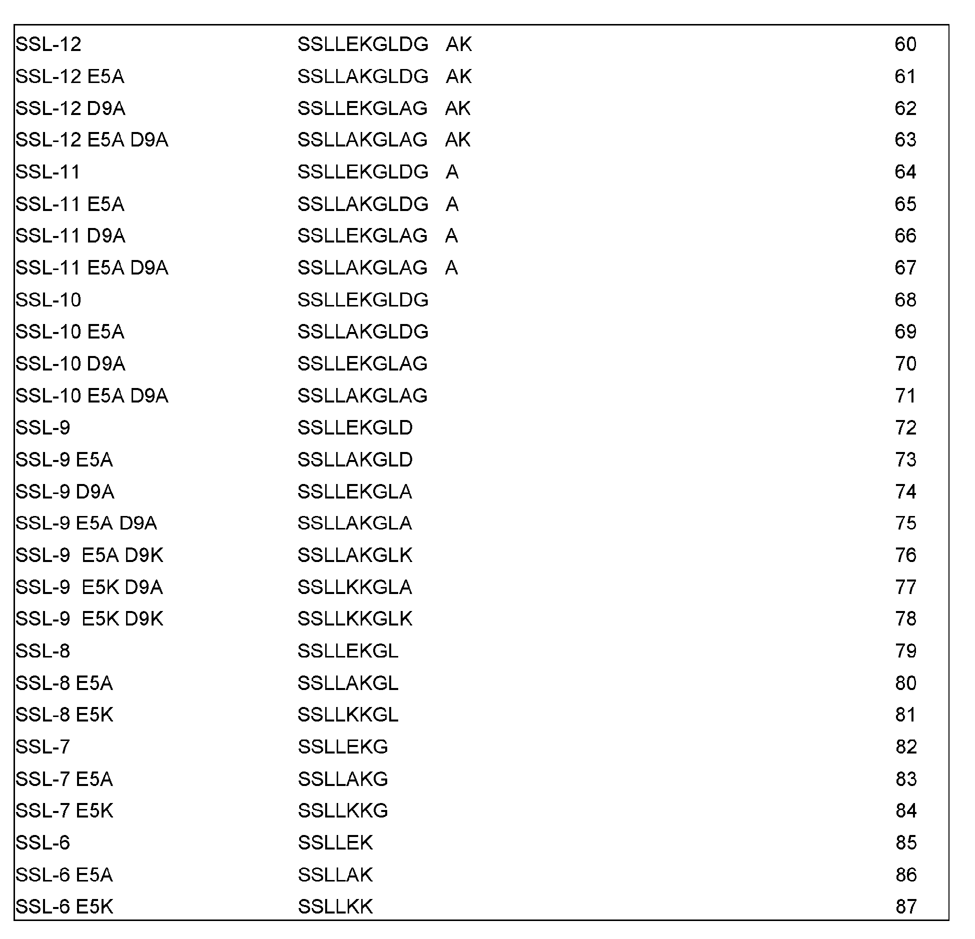

As defined below, the DDPs of the present invention as shown in the above SEQ-ID's 1 to 87 further include respective retro-, inverso- and retroinverso-forms thereof, and derivatives and analogues derived therefrom (as defined below).

In one aspect of the present invention the dermcidin-derived peptides (DDPs) are selected from the group consisting of DCD-1L, DCD-1, SSL-46, SSL-45, SSL-29, and SSL-25 to SSL-13 and the respective charge mutants thereof, having the amino acid sequences from SEQ ID No. 1 to SEQ ID No. 59 according to Table 2 above, including retro-, inverso- and retroinverso-forms thereof, or derivatives and analogues derived therefrom (as defined below).

In a further aspect of the present invention the dermcidin-derived peptides (DDPs) are selected from the group consisting of DCD-1L, DCD-1, SSL-46, SSL-45, SSL-29, SSL-25, SSL-25 E5A, SSL-25 D9A, SSL-25 D24A, SSL-25 E5A D9A D24A, SSL-23, SSL-21, SSL-19, SSL-17, SSL-15, SSL-13, having the amino acid sequences SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 15, SEQ ID No. 17, SEQ ID No. 19, including retro-, inverso- and retroinverso-forms thereof, or derivatives and analogues derived therefrom (as defined below).

In a further aspect of the present invention the dermcidin-derived peptides (DDPs) are selected from the group consisting of DCD-1L, DCD-1, SSL-46, SSL-45, SSL-29, SSL-25, having the amino acid sequences SEQ ID No. 1, SEQ ID No. 2, SEQ ID No. 3, SEQ ID No. 4, SEQ ID No. 5, SEQ ID No. 6, including retro-, inverso- and retroinverso-forms thereof, or derivatives and analogues derived therefrom (as defined below).

In a further aspect of the present invention the dermcidin-derived peptides (DDPs) are DCD-1L (SEQ ID No. 1) and SSL-25 (SEQ ID No. 6), including retro-, inverso- and retroinverso-forms thereof, or derivatives and analogues derived therefrom (as defined below).

In a further aspect of the present invention the dermcidin-derived peptides (DDPs) are selected from the group consisting of SSL-13 to SSL-6 and the respective charge mutants thereof, having the amino acid sequences from SEQ ID No. 56 to SEQ ID No. 87 according to Table 2 above, including retro-, inverso- and retroinverso-forms thereof, or derivatives and analogues derived therefrom (as defined below).

In a further aspect of the present invention the dermcidin-derived peptides (DDPs) are selected from the group consisting of SSL-13, SSL-13 D9A, SSL-11, SSL-11 E5A D9A, SSL-9, SSL-9 E5A D9K, SSL-9 E5K D9A, SSL-9 E5K D9K, having the amino acid sequences SEQ ID No. 56, SEQ ID No. 58, SEQ ID No. 64, SEQ ID No. 67, SEQ ID No. 72, SEQ ID No. 76, SEQ ID No. 77, SEQ ID No. 78, including retro-, inverso- and retroinverso-forms thereof, or derivatives and analogues derived therefrom (as defined below).

In a further aspect of the present invention the dermcidin-derived peptides (DDPs) are selected from the group consisting of SSL-24, SSL-24 E5A, SSL-24 D9A, SSL-24 D24A, SSL-24 E5A D9A D24A, SSL-23 E5A, SSL-23 D9A, SSL-23 E5A D9A, SSL-22, SSL-22 E5A, SSL-22 D9A, SSL-22 E5A D9A, SSL-21 E5A, SSL-21 D9A, SSL-21 E5A D9A , SSL-20, SSL-20 E5A, SSL-20 D9A, SSL-20 E5A D9A, SSL-19 E5A, SSL-19 D9A, SSL-19 E5A D9A, SSL-18, SSL-18 E5A, SSL-18 D9A, SSL-18 E5A D9A, SSL-17 E5A, SSL-17 D9A, SSL-17 E5A D9A, SSL-16, SSL-16 E5A, SSL-16 D9A, SSL-16 E5A D9A, SSL-15 E5A, SSL-15 D9A, SSL-15 E5A D9A, SSL-14, SSL-14 E5A, SSL-14 D9A, SSL-14 E5A D9A, SSL-13 E5A, SSL-13 D9A, SSL-13 E5A D9A, SSL-12, SSL-12 E5A, SSL-12 D9A, SSL-12 E5A D9A, SSL-11, SSL-11 E5A, SSL-11 D9A, SSL-11 E5A D9A, SSL-10, SSL-10 E5A, SSL-10 D9A, SSL-10 E5A D9A, SSL-9, SSL-9 E5A, SSL-9 D9A, SSL-9 E5A D9A, SSL-9 E5A D9K, SSL-9 E5K D9A, SSL-9 E5K D9K, SSL-8, SSL-8 E5A, SSL-8 E5K, SSL-7, SSL-7 E5A, SSL-7 E5K, SSL-6, SSL-6 E5A, SSL-6 E5K, having the amino acid sequences as indicated in Table 2 above, including retro-, inverso- and retroinverso-forms thereof, or derivatives and analogues derived therefrom (as defined below).

In a further aspect of the present invention the dermcidin-derived peptides (DDPs) are selected from the group consisting of SSL-13 D9A, SSL-11, SSL-11 E5A D9A, SSL-9, SSL-9 E5A D9K, SSL-9 E5K D9A, SSL-9 E5K D9K, having the amino acid sequences SEQ ID No. 58, SEQ ID No. 64, SEQ ID No. 67, SEQ ID No. 72, SEQ ID No. 76, SEQ ID No. 77, SEQ ID No. 78, including retro-, inverso- and retroinverso-forms thereof, or derivatives and analogues derived therefrom (as defined below).

In a further aspect of the present invention the dermcidin-derived peptides (DDPs) are selected from the group consisting of SSL-11, SSL-11 E5A, SSL-11 D9A, SSL-11 E5A D9A, SSL-10, SSL-10 E5A, SSL-10 D9A, SSL-10 E5A D9A, SSL-9, SSL-9 E5A, SSL-9 D9A, SSL-9 E5A D9A, SSL-9 E5A D9K, SSL-9 E5K D9A, SSL-9 E5K D9K, SSL-8, SSL-8 E5A, SSL-8 E5K, SSL-7, SSL-7 E5A, SSL-7 E5K, SSL-6, SSL-6 E5A, SSL-6 E5K, having the amino acid sequences as indicated in Table 2 above, including retro-, inverso- and retroinverso-forms thereof, or derivatives and analogues derived therefrom (as defined below).

In a further aspect of the present invention the dermcidin-derived peptides (DDPs) are selected from the group consisting of SSL-11, SSL-11 E5A D9A, SSL-9, SSL-9 E5A D9K, SSL-9 E5K D9A, SSL-9 E5K D9K, having the amino acid sequences SEQ ID No. 64, SEQ ID No. 67, SEQ ID No. 72, SEQ ID No. 76, SEQ ID No. 77, SEQ ID No. 78, including retro-, inverso- and retroinverso-forms thereof, or derivatives and analogues derived therefrom (as defined below).

The inventors have further found, that even higher translocation rates can be achieved by using peptides, wherein, starting from SSL-25 (SEQ ID No. 6), the positive net charge has successively been increased by replacing Asp or Glu, respectively, with Ala. Examples of such peptides are the above shown amino acid sequences SEQ ID No. 7, SEQ ID No. 8, SEQ ID No. 9, and SEQ ID No. 10, which can also be mentioned as a preferred group of peptides with particular cell-penetrating properties according to the present invention.

The inventors have further found, that similar results regarding higher translocation rates can be achieved by using peptides, wherein, starting from SSL-19 (SEQ ID No. 32), SSL-17 (SEQ ID No. 40), SSL-15 (SEQ ID No. 48), SSL-13 (SEQ ID No. 56), SSL-11 (SEQ ID No. 64) and SSL-9 (SEQ ID No. 72), the positive net charge has successively been increased by replacing Asp with Ala and/or Glu with Ala or Lys and/or Asp with Lys or Ala. Examples of such peptides are shown in Table 2 above, and which can also be mentioned as a preferred peptide with particular cell-penetrating properties according to the present invention.

Similar results regarding higher translocation rates can be expected by using peptides, wherein, starting from SSL-24 (SEQ ID No. 11), SSL-23 (SEQ ID No. 16), SSL-22 (SEQ ID No. 20), SSL-21, (SEQ ID No. 24), SSL-20 (SEQ ID No. 28), SSL-18 (SEQ ID No. 36), SSL-16 (SEQ ID No. 44), SSL-14 (SEQ ID No. 52), SSL-12 (SEQ ID No. 60), SSL-11 (SEQ ID No. 64) SSL-10 (SEQ ID No. 68), SSL-9 (SEQ ID No. 72), SSL-8 (SEQ ID No. 79), SSL-7 (SEQ ID No. 82) and SSL-6 (SEQ ID No. 85) the positive net charge is successively increased by replacing Asp with Ala and/or Glu with Ala or Lys and/or Asp with Lys or Ala. Examples of such peptides are shown in Table 2 above, which can also be mentioned as a preferred group of peptides with particular cell-penetrating properties according to the present invention.

Accordingly, in a further aspect of the present invention the dermcidin-derived peptides (DDPs) are selected from the group consisting of SSL-25 E5A, SSL-25 D9A, SSL-25 D24A, SSL-25 E5A D9A D24A, SSL-24 E5A, SSL-24 D9A, SSL-24 D24A, SSL-24 E5A D9A D24A, SSL-23 E5A, SSL-23 D9A, SSL-23 E5A D9A , SSL-22 E5A, SSL-22 D9A, SSL-22 E5A D9A, SSL-21 E5A, SSL-21 D9A, SSL-21 E5A D9A, SSL-20 E5A, SSL-20 D9A, SSL-20 E5A D9A, SSL-19 E5A, SSL-19 D9A, SSL-19 E5A D9A, SSL-18 E5A, SSL-18 D9A, SSL-18 E5A D9A, SSL-17 D9A, SSL-17 E5A D9A, SSL-16 E5A, SSL-16 D9A, SSL-16 E5A D9A, SSL-15 D9A, SSL-15 E5A D9A, SSL-14 E5A, SSL-14 D9A, SSL-14 E5A D9A, SSL-13 E5A, SSL-13 D9A, SSL-13 E5A D9A, SSL-12 E5A, SSL-12 D9A, SSL-12 E5A D9A, SSL-11 E5A, SSL-11 D9A, SSL-11 E5A D9A, SSL-10 E5A, SSL-10 D9A, SSL-10 E5A D9A, SSL-9 E5A, SSL-9 D9A, SSL-9 E5A D9A, SSL-9 E5A D9K, SSL-9 E5K D9A, SSL-9 E5K D9K, SSL-8 E5A, SSL-8 E5K, SSL-7 E5A, SSL-7 E5K, SSL-6 E5A, SSL-6 E5K, having the amino acid sequences as indicated in Table 2 above, including retro-, inverso- and retroinverso-forms thereof, or derivatives and analogues derived therefrom (as defined below).

Accordingly, in a further aspect of the present invention the dermcidin-derived peptides (DDPs) are selected from the group consisting of SSL-25 E5A (SEQ ID No. 7), SSL-25 D9A (SEQ ID No. 8), SSL-25 D24A (SEQ ID No. 9), SSL-25 E5A D9A D24A (SEQ ID No. 10), SSL-19 D9A (SEQ ID No. 34), SSL-17 D9A, (SEQ ID No. 42), SSL-15 D9A, (SEQ ID No. 50), SSL-13 D9A (SEQ ID No. 58), SSL-11 E5A D9A (SEQ ID No. 66), SSL-9 E5A D9K (SEQ ID No. 76), SSL-9 E5K D9A (SEQ ID No. 77), SSL-9 E5K D9K (SEQ ID No. 78), including retro-, inverso- and retroinverso-forms thereof, or derivatives and analogues derived therefrom (as defined below).

In a further aspect of the present invention the dermcidin-derived peptides (DDPs) are selected from the group consisting of SSL-13 E5A, SSL-13 D9A, SSL-13 E5A D9A, SSL-12 E5A, SSL-12 D9A, SSL-12 E5A D9A, SSL-11 E5A, SSL-11 D9A, SSL-11 E5A D9A, SSL-10 E5A, SSL-10 D9A, SSL-10 E5A D9A, SSL-9 E5A, SSL-9 D9A, SSL-9 E5A D9A, SSL-9 E5A D9K, SSL-9 E5K D9A, SSL-9 E5K D9K, SSL-8 E5A, SSL-8 E5K, SSL-7 E5A, SSL-7 E5K, SSL-6 E5A, SSL-6 E5K, having the amino acid sequences as indicated in Table 2 above, including retro-, inverso- and retroinverso-forms thereof, or derivatives and analogues derived therefrom (as defined below).

In a further aspect of the present invention the dermcidin-derived peptides (DDPs) are selected from the group consisting of SSL-13 D9A (SEQ ID No. 58), SSL-11 E5A D9A (SEQ ID No. 67), SSL-9 E5A D9K (SEQ ID No. 76), SSL-9 E5K D9A (SEQ ID No. 77), SSL-9 E5K D9K (SEQ ID No. 78), including retro-, inverso- and retroinverso-forms thereof, or derivatives and analogues derived therefrom (as defined below).

In a further aspect of the present invention the dermcidin-derived peptides (DDPs) are selected from the group consisting of SSL-11 E5A, SSL-11 D9A, SSL-11 E5A D9A, SSL-10 E5A, SSL-10 D9A, SSL-10 E5A D9A, SSL-9 E5A, SSL-9 D9A, SSL-9 E5A D9A, SSL-9 E5A D9K, SSL-9 E5K D9A, SSL-9 E5K D9K, SSL-8 E5A, SSL-8 E5K, SSL-7 E5A, SSL-7 E5K, SSL-6 E5A, SSL-6 E5K, having the amino acid sequences as indicated in Table 2 above, including retro-, inverso- and retroinverso-forms thereof, or derivatives and analogues derived therefrom (as defined below).

In a further aspect of the present invention the dermcidin-derived peptides (DDPs) are selected from the group consisting of SSL-11 E5A D9A (SEQ ID No. 67), SSL-9 E5A D9K (SEQ ID No. 76), SSL-9 E5K D9A (SEQ ID No. 77), SSL-9 E5K D9K (SEQ ID No. 78), including retro-, inverso- and retroinverso-forms thereof, or derivatives and analogues derived therefrom (as defined below).

Overall charge of the peptide is often essential for the initial binding of the peptide to the membrane and also plays an important role in non-covalent complex formation particularly when nucleic acid delivery is considered. Accordingly, the present invention also comprises the dermcidin-derived peptides (DDPs) DCD-1L, DCD-1, SSL-46, SSL-45, SSL-29, SSL-25, etc. as shown in Table 2 above, wherein the charge of the peptide has been conserved or altered, also being designated herein as "charge mutants". Such charge mutants can be obtained either by exchanging homologous amino acids, or by replacing any amino acid via a charged amino acid residue such as Glu, Asp, Lys, Arg or by an unnatural amino acids bearing a polar or charged side chain or by moving the charged residue within the sequence without changing the overall charge. The corresponding D-enantiomers of the above mentioned amino acids may also be used to produce such charge mutants which can be coupled to obtain an inverso-form as described below) and/or by replacing proteinogenic amino acids by other suitable non-proteinogenic amino acids ("analogues" as described below"). Such charge mutants are particularly preferred according to the present invention.

The inventors have further found, that even shorter peptides having less than 25 amino acids (i.e. being shorter than the above shown peptides of the SSL-25 series with SEQ ID Nos. 6 to 10), but still having equal to or more than 6 amino acids, are able to translocate. Examples of such peptides are the amino acid sequences shown in Table 2 above. These shorter peptides (C-terminal cleaved analogues of SSL-25), having < 25 and ≥ 6 amino acids in the peptide chain, are in particular preferred with respect to the more efficient preparation thereof.

A further aspect of the present invention relates to the further preferred very short peptides having < 15 and ≥ 6 amino acids in the peptide chain. Even more preferred are very short peptides according to the present invention having < 13 and ≥ 6 amino acids in the peptide chain.

Further aspects of the present invention relate to the above described very short peptides having < 25 and ≥ 8 amino acids in the peptide chain, having < 15 and ≥ 8 amino acids in the peptide chain, having < 13 and ≥ 8 amino acids in the peptide chain or having ≤ 11 and ≥ 8 amino acids in the peptide chain or having ≤ 11 and ≥ 8 amino acids in the peptide chain.

Further aspects of the present invention relate to the above described very short peptides having < 25 and > 8 amino acids in the peptide chain, having < 15 and > 8 amino acids in the peptide chain, having < 13 and > 8 amino acids in the peptide chain or having ≤ 11 and > 8 amino acids in the peptide chain or having ≤ 11 and > 8 amino acids in the peptide chain.

Such very short peptides are particularly preferred in view of the more cost effective preparation thereof. Further, the inventors of the present invention surprisingly found that very short peptides according to the present invention having < 13 and ≥ 8 amino acids in the peptide chain (i.e. having 12, 11, 10, 9 or 8 amino acids in the peptide chain), including their charge mutants, each as defined anywhere herein and as shown e.g. in Table 2 above are very efficient CPPs with no or marginal toxicity.

Thus, in accordance with the findings of the inventors the present invention relates to novel cell-penetrating peptides as a carrier for the transport of cargo molecules into cells, wherein the novel peptides are derived from the human dermcidin protein (SEQ ID No. 88) having a length ranging from 48 amino acids according to DCD-1L (SEQ ID No. 1) down to 6 amino acids, wherein peptides with a chain length ≥ 6 amino acids are derived from DCD-1L (SEQ ID No. 1) by cleavage from the C-terminal part and optionally amino acid substitution in the peptide chain. Preferably, the novel peptides of the present invention have a minimum chain length of 6, preferably 8, more preferably 9 amino acids. Preferably, the novel peptides of the present invention are characterized by the formula (I) or (Ia) as defined above.

In order to eliminate possible disadvantages of peptidic drugs, such as e.g. a potential low bioavailability due to proteolytic degradation, it is usual to replace some or all L-amino acids by their non-proteinogenic D-form. Accordingly, the present invention also comprises the dermcidin-derived peptides (DDPs) as defined above, such as DDPs according to the consecutive sequence of formula (I) or (Ia) above, e.g. DCD-1L, DCD-1, SSL-46, SSL-45, SSL-29, SSL-25, as well as those shown in Table 2 above, or the above described charge mutants thereof, wherein one or more L-amino acids are replaced by its corresponding D-form, also being designated herein as "inverso-form", as well as derivatives and analogues derived therefrom. For clarification it shall be mentioned that in the sense of the present invention the term "inverso-form" shall not be interpreted as restricted to the above sequences with complete replacement of all L-amino acids by their corresponding D-amino acids, but covers all of the sequences as shown above, wherein at least one L-amino acid has been replaced by its corresponding D-amino acid. Inverso-forms may also comprise charge mutants as mentioned above.

Likewise, it is also common to use reverse sequence (retro: interchanging of N and C-terminus) and accordingly, the present invention further comprises the dermcidin-derived peptides (DDPs) as defined above, such as e.g. DCD-1L, DCD-1, SSL-46, SSL-45, SSL-29, SSL-25, as well as those shown in Table 2 above, or the above described charge mutants thereof, in its reverse (retro) sequence form (i.e. with interchanged N- and C-terminus), also being designated herein as "retro-form", as well as derivatives and analogues derived including charge mutants therefrom.

According to the present invention, it is also possible to combine the replacement of one or more L-amino acids by one or more D-amino acids with a reverse sequence, such forms also being designated herein as "retroinverso-form".

Furthermore it is known that the properties of peptides often do not change significantly, if single or only few amino acids are exchanged homologously. Such a homologous exchange, provided that due to the exchange within one group the desired properties are not significantly altered, can be advantageous in cases where a homologously changed sequence can be prepared with higher yield. Such homologous amino acid may also be derived from existing non-natural amino acids which may also be of purely synthetic origin. Similarly, homologous peptides derived from skin or sweat of other mammal or animals other than human may show similar properties and can be respectively modified by known biotechnological techniques.

The invention further comprises analogues and derivatives of the described peptides. The term "analogue" of an amino acid sequence according to the present invention comprises in particular any amino acid sequence having a sequence identity of at least 85%, preferably at least 90%, more preferably at least 95%, and even more preferably of at least 99% identity to said sequence, and same or comparable properties or activity. Sequence identity can be determined either by common techniques based on peptide properties such as hydrophobic moment, charge, HPLC profile or by means of any computer tool generally used in the field. Examples comprise BLAST programs used with default parameters.

An analogue of an amino acid sequence of the invention may result from changes derived from mutation or variation in the sequences of peptides of the invention, including the deletion or insertion of one or more amino acids or the substitution of one or more amino acids by natural or unnatural analogues, or even to alternative splicing. Analogues may also comprise charge mutants as mentioned above. Several of these modifications may be combined. Preferably, an analogue of an amino acid sequence of the invention comprises conservative substitutions relative to the sequence of amino acids. Replacements of L-amino acids by non-natural amino acids such as N-methylated derivatives or amino acid homologs (e.g. Orn Dap, Dab instead of Lys) are common examples and are accordingly covered by the term "analogues of the amino acid sequences" according to the present invention. The present invention also comprises the dermcidin-derived peptides as defined above, such as e.g. DCD-1L, DCD-1, SSL-46, SSL-45, SSL-29, SSL-25, as well as those shown in Table 2 above, or the above described charge mutants thereof, wherein one or more L-amino acids are replaced by their homo- and nor-forms. The prefix "nor" refers to a structural analog that can be derived from a parent compound by the removal of one carbon atom along with the accompanying hydrogen atoms. The prefix "homo" indicates the next higher or lower member in a homologous series.

Likewise, well documented methods to enhance the bioavailability of the dermcidin-derived peptides as defined above, such as e.g. DCD-1L, DCD-1, SSL-46, SSL-45, SSL-29, SSL-25, as well as SSL-23, SSL-21, SSL-19, SSL-17, SSL-15, SSL-13, SSL-12, SSL-11, SSL-10, SSL-9, SSL-8, SSL-7 and SSL-6, or the above described charge mutants thereof, using standard methods of formulation, well-known excipients or using PEGylation and/or acetylation of side chains, cyclization, lipidation, stapling, or by use of enzyme inhibitors along with the peptides of the present invention are accordingly covered by the term "analogues of the amino acid sequences according to the present invention".

A dermcidin-derived peptide (DDP) analogue according to the invention can also be a peptidomimetic. A peptidomimetic is a cell-penetrating, functional protein-mimicking peptide which may be composed of β-peptides or peptoids or a peptide containing unnatural amino acids such as N-methylated or D-amino acids, such as in particular a peptide or peptidomimetic that will, due to its internal cell penetrating capacity, be internalized into a host cell, and once inside the host cell, will display a mimicking activity of either the original protein or peptide that it has been generated from, or a protein or peptide of choice to that it has been designed to mimic. A cell-penetrating, functional protein-mimicking peptide is thus defined as a cell-penetrating peptide that in itself has effector activity and that will activate or inactivate an internal and/or external signaling pathway and/or cascade, resembling the activated functional protein that it is derived from. It is therefore characterized as having both cellular penetrating capability and effector and/or functional protein-mimicking activity. A cellular effector can herein be either an intracellular and/or extracellular effector and is in the present context defined as a structure that produces a cellular effect, such as a contraction, secretion, electrical impulse, or activation or inactivation of an intracellular and/or extracellular signaling cascade, or that induces the up regulation of a cellular level of an mRNA and/or a protein, in response to a stimulation by said effector. A typical effector is in the present context selected from the group consisting of a metabolite, an antagonist, an agonist, a receptor ligand, a receptor coupled protein, an activated receptor, an enzyme inhibitor, activator / inactivator and/or stimulator, a kinase, a phosphatase, an enhancer, or a silencer such as siRNA, a transcription factor, a transporter and/or a transmitter, a hormone, a channel, an ion, a prion, and a viral protein.

Accordingly, the term "dermcidines", dermcidin-derived peptide (DDP) or dermcidin-derived peptides (DDPs) according to the present invention further comprises retro-, inverso- and retroinverso-forms of the sequences described herein, as well as analogues and derivatives, peptides with a homologous amino acid sequence, or peptidomimetics, which in any case exhibit comparable cell-penetrating properties according to the present invention.

The DDPs of the present invention can be prepared by conventional methods as known from the state of the art.

Dermcidin derived peptides (DDPs) utilized here can be obtained and isolated from natural sources namely human sweat, or can be expressed recombinantly or chemically synthesized. In large amounts, dermcidin derived peptides can be obtained by well-known solid phase peptide synthesis protocols based on Merrifield method [R. B. Merrifield, Science (1965) 150, 178-85]. In this method, the peptide chain is covalently attached to the polymeric solid support at the C-terminus end of the desired peptide and the peptide chain is elongated by removing the deprotection group at the amino-terminus followed by coupling of the next amino acid. This cycle is repeated until the entire length of the peptide is obtained on the solid support. Suitable orthogonal protection is utilized for cysteine, polar, basic, cyclic and aromatic amino acids which are removed in the last step of removing the fully synthesized peptides from the solid support. Well established molecular biology protocols to express polypeptides through the DNA sequences encoding the target sequence are also available to obtain dermcidin derived peptides where the expression can be performed in insect cells, bacterial cells, plant cells, fungi and yeast cells in addition to mammalian cell culture. Recombinant synthesis of DCD in *E. coli* is previously reported [I. Čipáková, J. Gašperik, E. Hostinová, Expession and purification of human antimicrobial peptide dermcidin in Escherichia coli, Protein Expression and Purification (2006) 45, 269-274]. Using these expression methods, it is possible to express an optimized dermcidin derived peptide sequence which could be fused to peptide, peptoid, PNA, antimicrobial, fusogenic, cosmetically or pharmaceutically relevant drug with or without any cleavable linker to be administered or delivered in monomeric or in multimeric form into the target cells determined by the conjugated drug.

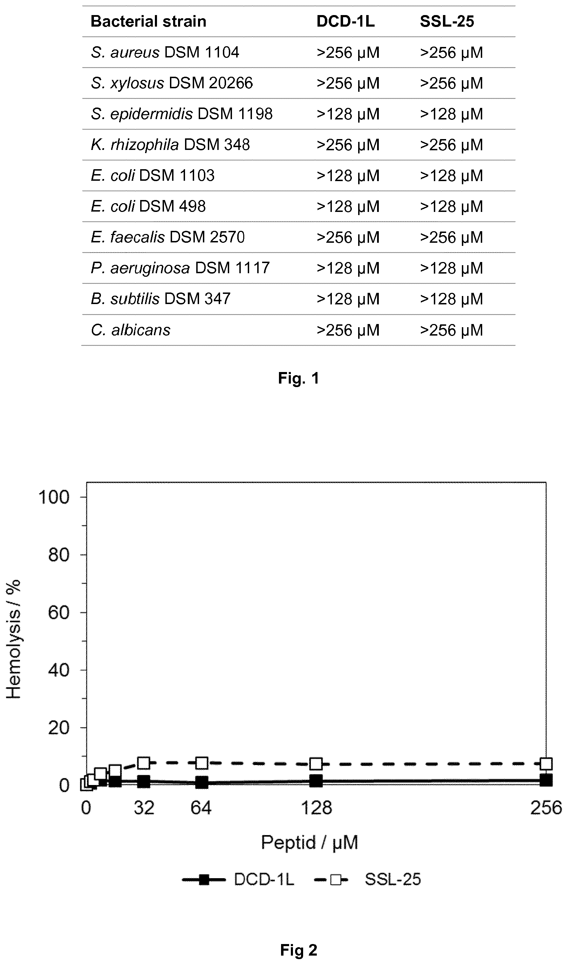

As mentioned above, the inventors could not detect an antimicrobial activity for several bacterial strains, neither for DCD-1L nor for SSL-25. As shown in detail in Example 1 below, both peptides do not effect an inhibition of bacterial growth. Typical inhibition concentrations of antimicrobial peptides range between 1 to 10 µM and in the present experiments no antibacterial growth occurred even with concentrations up to 256 µM as shown in Figure 1.

As also mentioned above, the inventors could exclude the undesired side-effect of conventional antimicrobial peptides such as damage of eukaryotic membranes. As shown in detail in Example 2 below, no damage of the membrane of human erythrocytes in a hemolytic test assay occurred with DCD-1L and only marginal damage occurred with SSL-25 as shown in Figure 2. Accordingly, the DDPs of the present invention are not toxic for eukaryotic cells.

The results of the above cited scientific publication (*Steffen et al. 2006*) confirm that despite their amphiphilic structure dermcidin peptides do not effect permeabilization of bacterial membranes or lipid vesicles. However, it has been described that DCD-1L forms pores in lipid membranes [C. Song, C. Weichbrodt, E.S. Salnikov, M. Dynowski, B.O. Forsberg, B. Bechinger, C. Steinem, B.L. de Groot, U. Zachariae, K. Zeth, Crystal structure and functional mechanism of a human antimicrobial membrane channel, P Natl Acad Sci USA, 110 (2013) 4586-4591]. Said pore formation occurred only in the presence of Zn²⁺ ions. The inventors of the present invention now examined, whether Zn²⁺ ions might be of essential importance for the antimicrobial function of the peptides. In the Examples below, a possible influence of Zn²⁺ has thus been considered, also in the antimicrobial evaluation of Example 1.

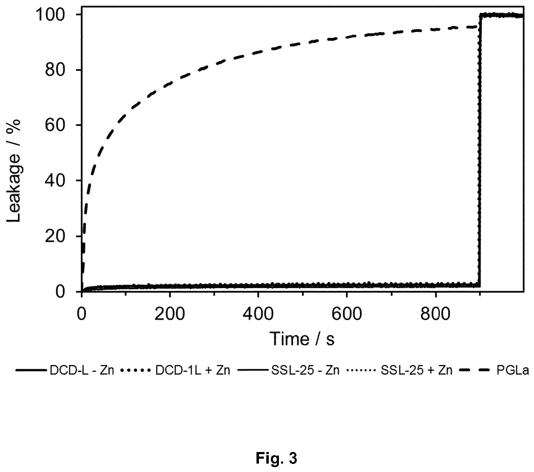

The investigation of a possible formation of pores in lipid membranes by the DDPs of the present invention has been carried out as described in detail in Example 3 using lipid vesicles. Therein, the leakage of a fluorescent dye incorporated in the vesicles after the addition of the dermcidin-derived peptides of the present invention over a period of 15 minutes has been examined. Both, DCD-1L and SSL-25 led only to extremely small detectable signals which did not significantly increase even when 100 µM Zn²⁺ ions were added, as shown in Figure 3. In all cases the leakage of not more than 2 to 3 % was marginal so that this cannot be considered as any noticeable membrane damage. According to the above mentioned publication (Song et. al, 2013) a potential across the membrane promotes pore formation and in consequence thereof, leakage would occur. However, even after applying a potential of 100 mV no significant change in the leakage was observed irrespective of the polarity of the potential or of the presence of Zn²⁺ ions. In contrast, already 10 times lower concentrations of a typical pore forming antimicrobial peptide such as PGLa, effects such a serious membrane damage that within a few minutes an almost complete efflux of the entrapped fluorescent dye (leakage) takes place, as also shown in Figure 3. These experiments confirm that the lipid membrane (in vesicles or in live bacteria from Figure 1) cannot be perturbed by DCD-1L or by SSL-25 and may explain the missing antimicrobial activity of the dermcidines of the present invention.

In contrast, the results of the Examples 4 to 8 as shown in detail below clearly and unambiguously demonstrate the translocation properties of the dermcidin peptides of the present invention.

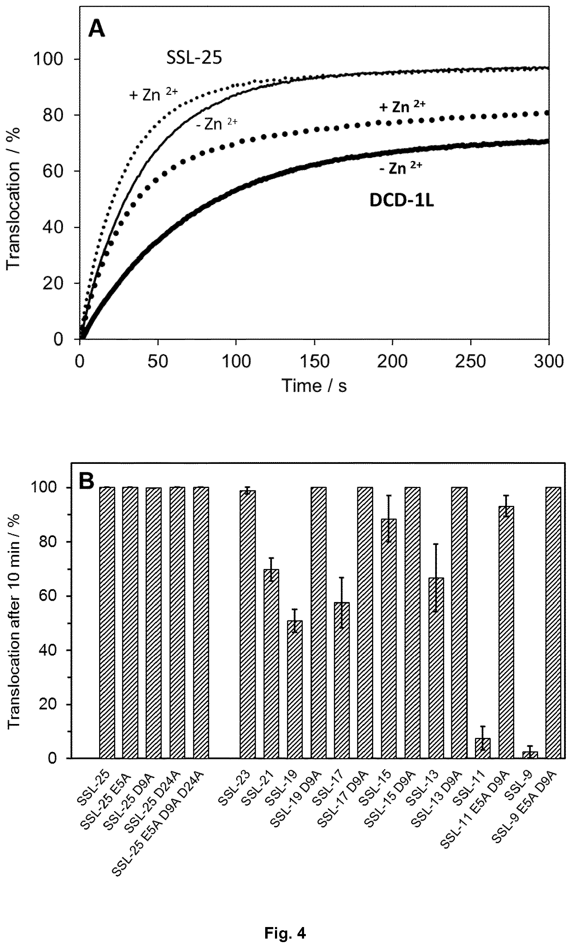

In Example 4 as described in detail below in a test assay according to *Marks et al. (2011)* a nearly complete translocation of the peptides DCD-1L and SSL-25, respectively, into lipid vesicles, i.e. through a hydrophilic barrier, could be observed within 3 to 5 minutes, as shown in Figure 4A. The presence of Zn²⁺ ions further accelerated this translocation effect. However, the efficiency of translocation was not improved by other divalent ions such as Ca^{++,} Mg⁺⁺, Co⁺⁺ or Ni⁺⁺ and therefore, this effect seems to be specific for Zn⁺⁺ ions. In Figure 4B it is shown the extent of translocation of SSL-25 and its analogues (without Zn²⁺ ions) achieved after 10 minutes.

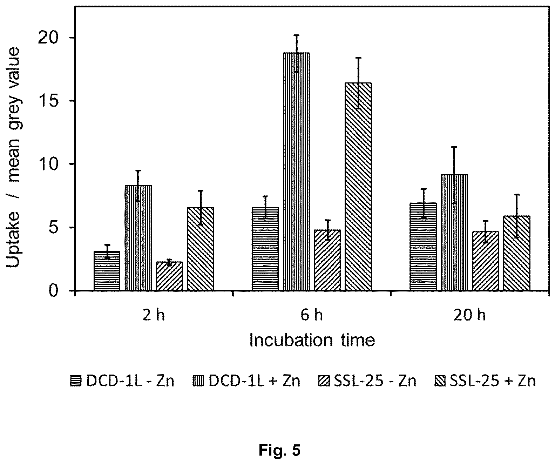

In Example 5 as described in detail below the translocation of the peptides DCD-1L and SSL-25 in eukaryotic cells has been shown in BY2 tobacco cells. Therein, the translocation has been examined on DCD-1L and SSL-25 N-terminally labeled with rhodamine B by using fluorescence microscopy. The results are shown in Figure 5. A comparison of the pictures, taken after 2 h, 6 h and 20 h leads to the conclusion that a relative maximum of the translocation is achieved after 6 h. Further, it can be seen that the presence of Zn²⁺ ions increases the translocation efficiency.

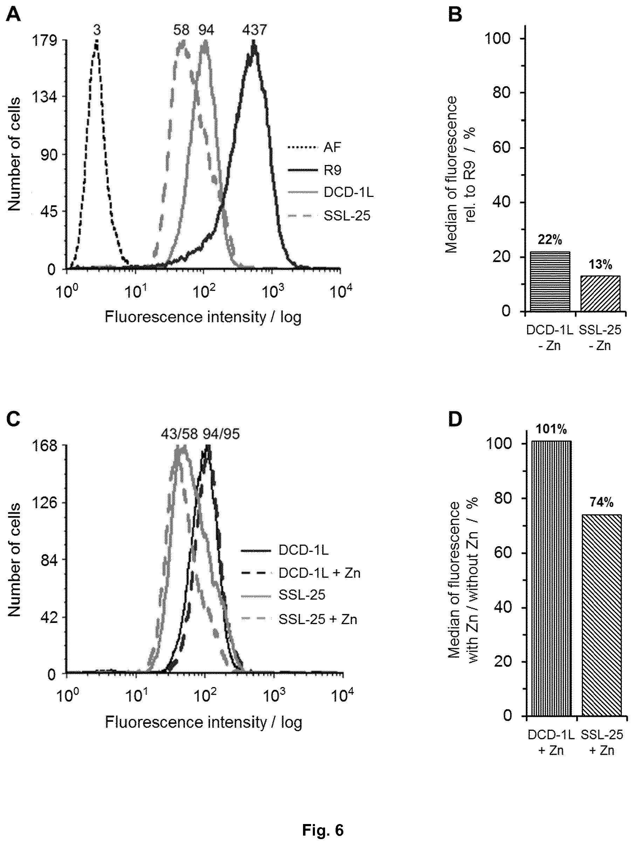

Further, in Example 6 as described in detail below the translocation of the peptides DCD-1L and SSL-25 in human HeLa-cells has been shown. Therein, the peptides have been labeled N-terminally with carboxyfluorescein and its translocation was demonstrated by using Fluorescence-activated Cell Sorting (FACS), as shown in Figure 6. Therein, both peptides turned out to be very effective, because - compared to the reference CPP R9 - already after 2 hours the cells incorporated 23 % of DCD-1L and 13 % SSL-25, respectively.

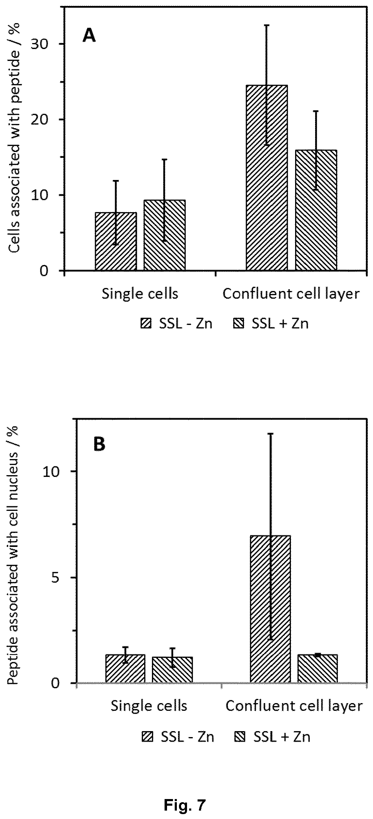

To further investigate the translocation of the dermcidin-derived peptides of the present invention into skin cells, in Example 7 as described in detail below HaCaT-keratinocytes, an immortalized non malignant cell line [P. Boukamp, R.T. Petrussevska, D. Breitkreutz, J. Hornung, A. Markham, N.E. Fusenig, Normal Keratinization in a Spontaneously immortalized Aneuploid Human Keratinocyte Cell-Line, J Cell Biol, 106 (1988) 761-771] has been used. Therein, the cellular uptake of SSL-25 N-terminally labeled with rhodamine B in a concentration of 2 µM, has been evaluated. The cells were present both as single cells and as a confluent cell layer. Using fluorescence microscopy already after 15 minutes a significant translocation could be observed, which was more distinct in the cell layer (25 %) than in the individual cells (8 %), as shown in Figure 7A. A certain amount of the peptide was associated with the cell nucleus (2 to 7 %) as shown in Figure 7B.

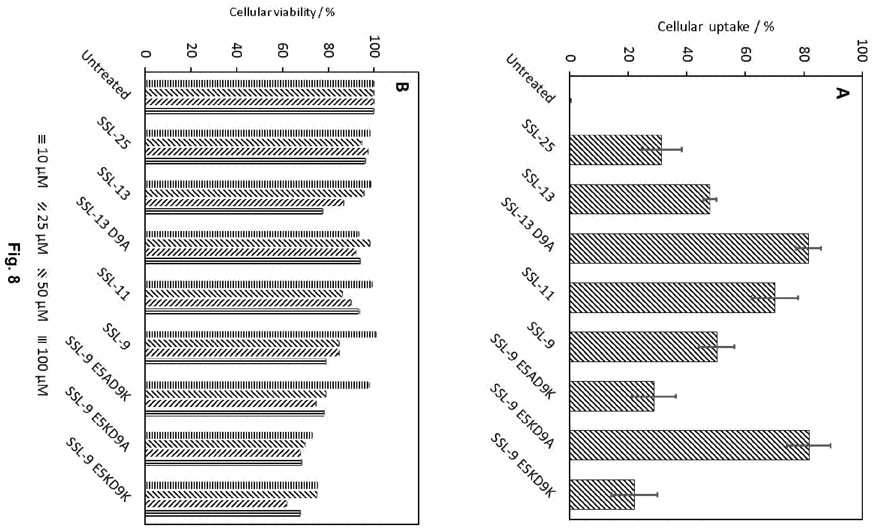

In Example 8, the translocation and cytotoxicity of shorter analogues of SSL25 (SSL-13, SSL-11 and SSL-9) and their charge mutants (SSL-13 D9A, SSL-9 E5A D9K, SSL-9 E5K D9A and SSL-9 E5K D9K) in HaCaT- Keratinocyte cell line has been shown with reference to SSL-25. Therein, the peptides were labeled with carboxyfluorescein at the N-terminus and their translocation was determined by flow cytometry, as shown in Figure 8A. Therein, the shorter peptides SSL-13, SSL-11 and SSL-9 and the charge mutants, SSL-13 D9A and SSL-9 E5K D9A, show higher cell uptake in comparison to SSL-25 after 1 h incubation at a concentration of 25µM whereas SSL-9 E5A D9K and SSL-9 E5K D9K show reduced but comparable uptake. Furthermore, after 24 h incubation of cells with these set of peptides, at least 70% cell viability was maintained in presence of all peptides up to a concentration of 100µM as shown in Figure 8B. These results lead to the conclusion that shorter analogues of SSL-25 and their charge mutants used here are able to penetrate HaCaT-keratinocyte cells in 1h without causing significant toxicity.

The results of these Examples 1 to 8 confirm the surprising object of the present invention, and accordingly, in a first aspect, the present invention relates to the use of at least one dermcidin-derived peptide as defined above as a cell-penetrating agent.

As shown in several of the Examples, the cell-penetrating effect can be accelerated or increased in the presence of Zn²⁺ ions, and accordingly in a further aspect the peptides of the present invention are used in the presence of Zn²⁺ ions. The Zn²⁺ ions are then added in an amount sufficient to accelerate or increase the translocation of the cell-penetrating dermcidin-derived peptide into the cells. Suitable amounts of Zn²⁺ ions can be selected from a range of 1 µM to 1 mM. Preferably Zn²⁺ ions are added or present in an amount / concentration of 100 µM where the cells do not show any observable toxicity.

Suitable sources of Zn²⁺ ions should be able to release Zn²⁺ ions in an amount sufficient to achieve the accelerating effects as described above and should further not harm the biological or physiological system, wherein they are used. They should in particular not be toxic. Typical sources of Zn²⁺ ions are Zn-salts, Zn-complexes, nano-Zn, metallic Zn, etc.

In view of the findings of the inventors of the present invention, the peptides as defined herein can be considered as useful for the transport of pharmaceutically, cosmetically or biotechnologically interesting substance or bioactive molecules into cells, in particular into the cytoplasmic, mitochondrial and nuclear compartments of a living cell and/or microorganism, without disrupting the plasma membrane, as well as for delivering bioactive molecules, in particular polar or hydrophilic macromolecules, across the blood-brain barrier, permitting e.g. the intracellular transport of conjugated bioactive molecules, such as oligopeptides and oligonucleotides and drugs. Accordingly, in the sense of the present invention the term "cell-penetrating properties" (similarly "cell-penetrating capacity") relates to the capability of the dermcidin-derived peptides as described herein to translocate across the plasma membrane into either cytoplasmic and/or nuclear compartments of eukaryotic (and/or prokaryotic) cells, such as into cytoplasm, nucleus, lysosome, endoplasmic reticulum, Golgi apparatus, mitochondria and/or chloroplast, seemingly energy-independently. Additionally, the term "cell-penetrating capacity" of a peptide of the present invention can in some aspects of the invention also be used synonymously to indicate transcellular or transmembrane transport, and thus also stand for e.g. the capability to translocate across an epithelial membrane, such as across the epithelium in the intestinal/buccal system, the mucosa in the mouth, lung, rectum or nose, or the blood-brain barrier of a mammal. The DDPs are thus further suitable for the transport of the cargo molecules into tissue and organs.

Accordingly, in the sense of the present invention a dermcidin-derived peptide is found to exhibit "cell-penetrating properties" ("cell-penetrating capacity") or to be effective as a cell-penetrating agent when having a capability to translocate as defined above.

The suitability of a dermcidin-derived peptide as a cell-penetrating agent or its capability to translocate can be determined with any (one or more) of the tests or assays as described in the present application.

Preferably, the peptides according to the present invention are considered to have "cell-penetrating properties" or have "cell-penetrating capacity" ("cell-penetration efficacy") if a cellular translocation (cellular penetration or cellular uptake) of ≥ 5,0 %, preferably ≥ 7.0 %, more preferably ≥ 10,0 %, is determined under the test conditions as described in Example 8 below, but independent of cell-types to be penetrated, independent of incubation time and independent of the cargo-concentration. More preferably the above defined efficient translocation percentages are determined under the test conditions as described in Example 8 below.

In general, cellular delivery using a cell-penetrating peptide and/or an analogue thereof as defined herein is non-invasive, energy-independent, efficient for a broad range of cell types and/or a broad variety of cargo molecules, applicable to cells *en masse,* non-saturable, and/or receptor independent.

Therewith, the cell-penetrating dermcidin-derived peptides (DDPs) of the present invention are suitable agents for the use in cosmetic applications as well as in medical and diagnostic applications or as a cosmetic or medical marker or as a part of a kit which performs transfection or diagnosis. Generally the term "marker" may comprise genetic, biological, biotechnological and molecular marker or any other peptide exhibiting a marker function. Due to the shown cell-penetrating properties the peptides are further suitable in biotechnological applications as well as a research tool for delivering e.g. tags and markers and/or for changing membrane potentials and/or properties.

The cell-penetrating dermcidin-derived peptides can be used *in vivo* as well as *in vitro.*

Accordingly, the dermcidin-derived peptides of the present invention can be used as a pharmaceutical, diagnostic, cosmetic or biotechnological agent or as a cosmetic or medical marker, for example as a marker in tumor diagnostic and tumor therapy, being based on the newly found underlying mechanism of the cell-penetrating properties of the peptides of the present invention or by adding specific markers (for example tumor specific markers) or recognition units to dermcidin derived peptides to promote targeted delivery (for example to cancer cells). Therewith, the peptides exhibit particular suitability for the use as a carrier for the transport of bioactive substances, so-called cargo molecules, through lipid membranes, i.e. in particular into cells.

According to the present invention, the cargo molecules may be selected from any pharmaceutically, cosmetically or biotechnologically interesting substance or bioactive molecule, including in particular pharmaceutically active substances (drug substances), cosmetically active substances and diagnostic substances as well as biotechnology agents. In a particular embodiment the cargo molecules are selected among hydrophilic macromolecules.

According to the present invention, the cargo molecules may be selected from the group consisting of polymers, such as in particular hydrophilic polymers, nanoparticles, peptides, polypeptides, proteins, antibodies, recombinant proteins, small molecular substances, nucleic acids, mononucleotides, oligonucleotides, polynucleotides, antisense-molecules, double stranded or single stranded DNA, RNA, antisense-RNA molecules, siRNA molecules, molecular active agents, pharmaceutically active agents, cosmetically active agents, diagnostic agents, dyes and contrast agents or transfection reagents attached covalently or complexed non-covalently.

Preferably, the cargo molecule is selected from the group consisting of peptides, proteins, nucleic acids, hydrophilic polymers, nanoparticles, molecular active agents, pharmaceutically active agents (i.e. drugs), cosmetically active agents, diagnostic agents, biotechnologically active agents, fluorescent and NIR-dyes, and contrast agents or small molecule drugs that can be covalently conjugated or non-covalently complexed.

In a further preferred embodiment the cargo molecule is selected among polar molecules.

In a further embodiment, the cargo molecule is selected among polypeptides and/or oligonucleotides with molecular weights several times greater than those of the dermcidin-derived carrier peptide.

The pharmaceutically, cosmetically, biotechnologically active substances and diagnostic substances include intracellular and extracellular substances. The dyes and contrast agents include in particular fluorescent and NIR-dyes.

The cargo molecules may generally be selected among active substances for treating mammals, including humans and animals.

The dermcidin-derived peptides of the present invention may generally be used for the transport of cargo molecules into cells selected from the group consisting of eukaryotic cells, prokaryotic cells, tumor cells, skin cells such as keratinocytes, plant cells, bacterial cells, etc.

In a further aspect of the present invention, the dermcidin-derived peptides (DDPs) according to the present invention can be used for coupling, either at their N-terminus, at their C-terminus or at a side chain residue, to a cell-penetrating peptide (CPP) different than those according to the present invention, such as e.g. an existing or conventional CPP or cell penetrating agents, for example those CPPs as described in the prior art and as mentioned above, or derivatives thereof (hereinafter also designated in summary as "CPP" or "CPPs"). This may be advantageous, if such a (different or conventional) CPP or derivative thereof has toxic or immunogenic side effects and can thus not be used as a CPP alone. The coupling of the human derived DPP of the present invention may decrease the toxic or immunogenic response of the CPP or derivative thereof, thus making such a CPP-DDP conjugate suitable for the use in the treatment of viable cells or in human or animal treatment. The coupling of the DDPs of the present invention to a CPP or derivative thereof may also enhance the cell penetration properties of both, the DDP and/or the CPP or derivative thereof. Thus, a further aspect of the present invention relates to the use of the DDPs of the present invention as a coupling agent or immune suppressing agent to CPPs (other than those of the present invention) or derivatives thereof as well as to the combination of DDP as defined herein and the coupled CPP or derivative thereof, thus forming a "CPP-DDP-complex" or "CPP-coupled DDP".

Particularly preferred cells to be penetrated *in vivo* or *in vitro* are selected from the group consisting of skin, cancer, nerve, kidney, plant, bacterial and possibly other 200 cell types present in the human body where the dermcidin-derived peptides do not show any toxicity when delivered alone or along with a drug or compound specific to a given cell type in form of any formulation, cream or oral supplement. Transfection of primary cells, of cell lines or cell cultures where nucleic acid sequence is to be administered dermcidin-derived peptides can be used as transfection reagents in above mentioned cell types. Likewise bacteria can be targeted via dermcidin-derived peptides as a vector to deplete or enhance their population, as well as yeasts, plants, fungi, etc.

A further aspect of the present invention relates to the combination of the cell-penetrating dermcidin-derived peptides (DDPs) as defined herein and one or more cargo molecule(s), as defined above, to be transported coupled with said DDP, thus forming a "cargo-coupled dermcidin-derived peptide" or "cargo-coupled DDP".

A further aspect of the present invention relates to the combination of a CPP-coupled DDP, as defined above, and one or more cargo molecule(s), as defined above, to be transported coupled with said CPP-coupled DDP, thus forming a "cargo-coupled CPP-DDP-complex". The coupling of the conventional CPP or derivative thereof to the DDPs according to the present invention may be carried out by conventional coupling techniques and well-known methods.

The cell-penetrating dermcidin-derived peptide (DDP) as defined herein and / or a cargo molecule to be transported coupled with said DDP or with said CPP-coupled DDP, thus forming a "cargo-coupled DDP" or "cargo-coupled CPP-DDP-complex" may be administered by various routes such as topical, enteral, oral, sublingual, inhalation or injection.

The cell-penetrating DDPs or the CPP-coupled DDPs according to the present invention may be coupled to a cargo molecule as defined herein for the transport into cells by conventional coupling techniques and well-known methods. There are a broad variety of methods for coupling a cargo to a cell-penetrating peptide, selected individually depending on the nature of cell-penetrating peptide, cargo and intended use.

In particular, coupling can be achieved via covalent and non-covalent binding. Examples of covalent coupling comprise direct covalent bond via a disulfide bridge (S-S-bridge), an amide bond or via other specific linker, as well as ester linkage, maleimide linker, succinimidyl ester, Isothiocyanate, click-chemistry based, chemical ligation, native chemical ligation, thiazodiline linker, aminooxy peptide linkage, hydrazinopeptide, thioether, thiomaleimide, etc. Examples of non-covalent coupling comprise complex formation, such as non-covalent coupling by electrostatic, ionic and/or hydrophobic interaction between CPP and cargo, or by adduct formation between the CPP and the cargo molecule. Examples of such non-covalent mode of cargo attachment have been reported in literature with well-known CPPs like Tat and Penetratin (Yuko Iwase, Noriyasu Kamei, EI-Sayed Khafagy, Mitsuko Miyamoto, Use of a non-covalent cell-penetrating peptide strategy to enhance the nasal delivery of interferon beta and its PEGylated form, International Journal of Pharmaceutics 510 (2016) 304-310]) Other examples for coupling via other specific linker(s) for antibody delivery may include an antibody linker which is advantageous with respect to its easy and flexible formation. For the transport of recombinant proteins, dermcidin-derived peptides (DDPs) of the present invention are also particularly suitable as "tag", which couples directly in the genetic sequence and can directly be expressed together with the sequence. This makes them particularly suitable in biotechnological applications.

In a further aspect of the invention the cell-penetrating dermcidin-derived peptides (DDPs) of the present invention may be coupled to a recognition unit, which is a peptide or amino acid sequence to which the DDP can be coupled, and which is specific to the desired target cell type. For example the peptide LTVSPWY may be used as tumor cell recognition unit, which would be able to promote targeted delivery of the DDP of the present invention to said target cells. A DDP coupled to a specific marker may also be designated as a "marker-coupled dermcidin-derived peptide". A DDP coupled to a specific recognition unit may also be designated as a "recognition unit-coupled dermcidin-derived peptide". It is further possible, that the DDP of the present invention may be coupled to a marker or recognition unit and to a cargo molecule as defined herein. In such twofold-coupled DDPs of the present invention the coupled marker or recognition unit provides a sequence tag such as tumor homing peptide [Pallavi Kapoor, Harinder Singh, Ankur Gautam, Kumardeep Chaudhary, Rahul Kumar, Gajendra P. S. Raghava, TumorHoPe: A Database of Tumor Homing Peptides; PLoS ONE 510 (2012), 7(4) e35187] which allows docking to the cell, e.g. here, the tumor cell, the DDP effects the translocation into the target cell, thus allowing the introduction of the coupled cargo molecule, e.g. an active ingredient, into the cell. Such marker-cargo-coupled (or recognition unit-cargo-coupled) DDPs may be particularly suitable as a pharmaceutical, diagnostic, cosmetic or biotechnological agent or as a cosmetic or medical marker. Preferably the marker or recognition unit and/or the cargo molecule are coupled to the DDP via a suitable linker. Preferably, a suitable linker provides a cleaving site for suitable enzymes such as lysosomal enzymes or β-glucuronidase, which effect cleavage of the coupled marker or recognition unit and/or cargo molecule inside the target cell. Likewise, nuclear delivery of a pharmaceutically relevant cargo molecule could be achieved by using a nuclear localization sequence (e.g. PKKKRKV) coupled to the dermcidin-derived peptides of the present invention.

Another aspect of the present invention relates to the use of the combination of a DDP as defined herein and a cargo molecule as defined herein, i.e. of a cargo-coupled DDP or of a cargo-coupled CPP-DDP-complex as defined herein, for the use as a pharmaceutical, cosmetic or diagnostic agent, as a medical or cosmetic marker, such as a marker in tumor diagnostic and tumor therapy, as well as a biotechnological agent.

The use of the cargo-coupled DDP or of the cargo-coupled CPP-DDP-complex may be carried out in the presence of Zn²⁺ ions, similarly as described above.

The invention further relates to a pharmaceutical composition comprising
i) at least one dermcidin-derived peptide (DDP) and/or at least one CPP-DDP-complex, each as defined above, with or without at least one cargo molecule as defined above; and/or
ii) at least one cargo-coupled DDP as defined above; and/or
iii) at least one cargo-coupled CPP-DDP-complex as defined above; and in each possible variation further
iv) optionally at least one further pharmaceutically active agent and/or at least one pharmaceutically acceptable solvent, auxiliary or excipient.

The invention further relates to a cosmetic composition comprising
i) at least one dermcidin-derived peptide (DDP) and/or at least one CPP-DDP-complex, each as defined above, with or without at least one cargo molecule as defined above; and/or
ii) at least one cargo-coupled DDP as defined above; and/or
iii) at least one cargo-coupled CPP-DDP-complex as defined above; and in each possible variation further
iv) optionally at least one further cosmetically active agent and/or at least one cosmetic solvent, auxiliary or excipient.

The invention further relates to a biotechnological composition comprising
i) at least one dermcidin-derived peptide (DDP) and/or at least one CPP-DDP-complex, each as defined above, with or without at least one cargo molecule as defined above; and/or
ii) at least one cargo-coupled DDP as defined above; and/or
iii) at least one cargo-coupled CPP-DDP-complex as defined above; and in each possible variation further
iv) optionally at least one further biotechnological agent and/or at least one biotechnologically acceptable solvent, auxiliary or excipient.

In a further aspect of the invention, similarly as described above, the aforesaid pharmaceutical, cosmetic or biotechnological composition may further comprise Zn²⁺ ions or a Zn²⁺ source, being suitable to release Zn²⁺ ions in an amount sufficient to achieve the accelerating effects as described above (for example ZnSO₄).

Another aspect of the present invention relates to a kit-of-parts combination comprising at least one dermcidin-derived peptide (DDP) and/or at least one CPP-DDP-complex, each as defined above, together with
i) at least one cargo molecule as defined above and/or
ii) Zn²⁺ ions or a Zn²⁺ source, as defined above
in a separated spatial arrangement.

Another aspect of the present invention relates to a kit-of-parts combination comprising at least one cargo-coupled dermcidin-derived peptide (cargo-coupled DDP) and/or at least one cargo- CPP-DDP-complex, each as defined above, together with Zn²⁺ ions or a Zn²⁺ source, as defined above, in a separated spatial arrangement.

The aforesaid kit-of-parts combinations may further comprise additional active agents, usual auxiliary substances, solvents and/or excipients, which are suitable for the intended use and application of said kit-of-parts combination.

Accordingly, the present invention also relates to a Kit-of-parts combination comprising
i) at least one dermcidin-derived peptide (DDP) as defined anywhere herein; and
ii) at least one cargo molecule as defined anywhere herein and/or
iii) Zn²⁺ ions or a Zn²⁺ source; and/or
iv) additional active agents, auxiliary substances, solvents and/or excipients, which are suitable for the intended use of said kit-of-parts combination
with the individual compounds being present in a separated spatial arrangement.

For clarifying reasons it should be noted that such kit-of-parts combination preferably comprises i) at least one DDP and ii) at least one cargo molecule, either in the form coupled to the DDP (i.e. in the form of a cargo-coupled DDP) or individually, e.g. in a spatial arrangement separated from the DDP.

The above mentioned kit-of-parts combinations may be composed with respect to the selection of DDP, CPP, cargo, Zn²⁺ ions / source, additional active agents, auxiliary substances, solvents and/or excipients to be suitable for use as a pharmaceutical, cosmetic, diagnostic or biotechnological kit.

A further aspect of the invention relates to the aforesaid pharmaceutical, cosmetic or biotechnological composition or the kit-of-parts combination for the use of transporting cargo molecules (as defined above) into cells.

The DDPs, CPP-DDP-complexes, cargo-coupled DDPs and/or cargo-coupled CPP-DDP-complexes, each as defined above, as well as the pharmaceutical compositions and pharmaceutical kits as described above may in particular be used in pharmaceutical applications for preventing, treating or ameliorating a condition or diseases of the skin or other tissues (melanoma, inflammation, infection, ageing).

A further aspect of the present invention relates to the peptides as described herein perse, according to the above given the consecutive sequence of formula (I) or (Ia) or (II) with the definition of X1 to X7 and Y1 to Y5 and Z1 as indicated above and having a maximum chain length of 19 amino acids in the peptide chain. Accordingly, the present invention in particular relates to peptides as in Table 2 above ranging from SSL-6 to SSL-19 and the respective charge mutants thereof, including retro-, inverso- and retroinverso-forms thereof,

### DESCRIPTION OF THE FIGURES

- Fig. 1: shows the results of the evaluation of the antimicrobial activity of DCD-1L and SSL-25 on various bacterial cultures.
- Fig. 2: shows the results of the evaluation of membrane damage of human erythrocytes by DCD-1L and SSL-25 in a hemolytic model.
- Fig. 3: shows the results of the evaluation of membrane damage in a lipid-vesicle leakage model.
- Fig. 4A: shows the results of the evaluation of translocation of DCD-1L and SSL-25 into lipid vesicles.
- Fig. 4B: shows the results of the evaluation of translocation of analogues of SSL-25 into lipid vesicles.
- Fig. 5: shows the results of the evaluation of translocation of DCD-1L and SSL-25 into BY2 tobacco cells.
- Fig. 6: A to D show the results of the evaluation of translocation of DCD-1L and SSL-25 into HeLa tumor cells.
- Fig. 7: A and B show the results of the evaluation of translocation of SSL-25 into human HaCaT keratinocytes.
- Fig. 8A,8B: show the results of the evaluation of cellular translocation and cytotoxicity of SSL-25, SSL-13, SSL-13 D9A, SSL-11, SSL-9, SSL-9 E5A D9K, SSL-9 E5K D9A, SSL-9 E5K D9K in HaCaT-Keratinocytes, respectively.

### EXAMPLES

### Example 1 Evaluation of Antimicrobial Activity

Cultures in early exponential growth of various bacterial species, as listed in table 3 below, were diluted (gram-positive strains 100-fold, gram-negative strains 1000-fold) and transferred into the cavities of microtiter plates in aliquots of 50 µl. The microtiter plates already contained the tested DDP (DCD-1L and SSL-25), also dissolved in 50 µl, in an increasing concentration series from 4 to 512 µM. After 20 hours to 22 hours in an incubator at 37 °C, 20 µl of a sterile 0.2 mg / ml resazurin solution were added into each cavity. A color change due to reduction of the reszurin directly indicates, whether the growth of the bacteria and thus the bacterial metabolism is inhibited by the DDPs or not. After additional 3 hours the color in the 96-well plates determines if the bacteria are live (pink) or dead (blue).

Table 3 (and Figure 1) shows the concentrations of DDP, which were required to effect a detectable inhibitory effect on the respective bacterial strain:

**Table 3:**

| **Bacterial strain** | **DCD-1**L | **SSL-25** |
|---|---|---|
| *S*. *aureus* DSM 1104 | >256 µM | >256 µM |
| *S*. *xylosus* DSM 20266 | >256 µM | >256 µM |
| *S. epidermidis* DSM 1198 | >128 µM | >128 µM |
| *K. rhizophila* DSM 348 | >256 µM | >256 µM |
| *E. coli* DSM 1103 | >128 µM | >128 µM |
| *E. coli* DSM 498 | >128 µM | >128 µM |
| *E. faecalis* DSM 2570 | >256 µM | >256 µM |
| *P. aeruginosa* DSM 1117 | >128 µM | >128 µM |
| *B*. *subtilis* DSM 347 | >128 µM | >128 µM |
| *C. albicans* | >256 µM | >256 µM |

Usual antimicrobial peptides inhibit bacterial growth already at concentrations 10 times or more below the concentrations as indicated in Table 3 and concentrations greater than 128 µM implies that peptides are unable to inhibit bacterial growth and hence are inactive Accordingly, the significantly higher concentrations detected herein for DCD-1L and SSL-25 clearly show, that the DDPs of the present invention cannot be considered as antimicrobials. With the present test results, no inhibition of the bacterial growth could be observed.

Even no inhibition could be detected by reducing the first incubation period from 20 h to 3 h.

Furthermore, neither in the case of *E*. *coli,* nor in the case of *S*. *aureus* an inhibitory effect could be detected when Zn²⁺ ions were present in the cultivation medium in a concentration of 1 mM.

As a result it can be summarized, that surprisingly the DDPs of the present invention, as shown on the exemplary peptides DCD-1L and SSL-25, are not active in the inhibition of the bacterial respiration and thus are no antimicrobial agents.

### Example 2 Evaluation of Membrane Damage in a Hemolytic Model in Human Erythrocytes

A concentrated solution of human erythrocytes was adjusted to a hematocrit of 0.5% by repeated washing with buffer solution (172 mM Tris-HCI, pH 7.6) and this diluted solution was equilibrated to 37 °C in a water bath.

The peptides were prepared as a 1024 µM stock solutions in buffer, added to the erythrocytes in sequential 2-fold dilutions and incubated at 37 °C for 30 minutes with occasional shaking.

The cells were centrifuged and the released hemoglobin in the supernatant was quantified using spectrophotometry. The obtained values were set in reference to the value obtained by complete lysis of all cells upon addition of Triton^{™} X-100. The results are shown in Figure 2.

Although the erythrocytes were present in the solution in strong dilution (0.25 % hematocrit) and no stabilizing additives such as BSA were present in the buffer, no hemolysis could be observed with DCD-1L and only a marginal hemolysis (< 10%) was observed with SSL-25.

These results show that the DDPs of the present invention, as shown on the exemplary peptides DCD-1L and SSL-25, did not or only to a negligible degree damage the erythrocyte membrane.

### Example 3 Evaluation of Membrane Damage in a Lipid-Vesicle Leakage Model

A damage of lipid membranes caused by peptides can be examined immediately and time-resolved via the leakage of a fluorescent dye encapsulated in lipid vesicles.

Large unilamellar lipid vesicles consisting of the lipids 1-palmitoyl-2-oleoyl-*sn*-glycero-3-phosphocholine (POPC) and 1-palmitoyl-2-oleoyl-*sn*-glycero-3-phospho-(1'-*rac*-glycerol) / (POPG) in a 70:30 molar ratio have been prepared, using known protocols, e.g Ellens et al. (1984) or in particular Steinbrecher et al. 2012 [H. Ellens, J. Bentz, F. C. Szoka.. pH-Induced destabilization of phosphatidylethanolamine-containing liposomes: role of bilayer contact. Biochemistry. 1984, 23,1532-1538 *;* T. Steinbrecher, S. Prock, J. Reichert, P. Wadhwani, B. Zimpfer, J. Buerck, M. Berditsch, M. Elstner, A.S. Ulrich, Peptide-lipid interactions of the stress-response peptide TisB that induces bacterial persistence, Biophys. J., 2012, 103, 1460-1469*].* The vesicles contained the fluorophore / quencher pair 8-aminonapthalene-1,3,6 trisulfonic acid (ANTS) / p-xylene-bis-pyridinium bromide (DPX). The increase of fluorescence due to leakage of the fluorophore ANTS was determined over a period of 15 min after peptide addition. After 900 sec the measurements were terminated by addition of Triton^{™} X-100 (0.3 % v/v) which dissolved the vesicles and yielded 100% leakage.

20 µM peptide were added to lipid vesicles (100 µM lipid) dissolved in buffer without or with 100 µM ZnSO₄. The results are shown in Figure 3.

Neither with SSL-25 nor with DCD-1L - regardless of the addition of Zn²⁺ ions (+ Zn / - Zn) - no noticeable leakage was observed, which leads to the conclusion that membrane damage does not occur. The same results were obtained if potentials of 100 mV and different polarity generated by valinomycin in combination with appropriate K⁺ - gradients had been applied, again showing no membrane damage. In contrast, upon addition of PGLa, an antimicrobial, pore forming peptide, a significant leakage (90% in 10 min) was observed already with a peptide concentration of 2 µM, i.e. 1/10 of the concentration of the DDPs of the present invention.

These results show that the DDPs of the present invention, as shown on the exemplary peptides DCD-1L and SSL-25, do not apparently damage lipid membranes.

### Example 4 Translocation of several peptides according to the invention into Lipid Vesicles

The translocation of dermcidin-derived peptides into lipid vesicles has exemplarily been demonstrated with DCD-1L and SSL-25 using the method described by Marks et al. [J.R. Marks, J. Piacone, K. Hristova, W.C. Wimley, Spontaneous Membrane-Translocating Peptides by Orthogonal High-Throughput Screening, J Am Chem Soc, 133 (2011) 8995-9004*].*

Similarly, the translocation of several dermcidin-derived peptides and charge mutants has been examines, including SSL-25, E5A, SSL-25 D9A, SSL-25 D24A, SSL-25 E5A D9A D24A, SSL-23, SSL-21, SSL-19, SSL-19 D9A, SSL-17, SSL-17, D9A, SSL-15, SSL-15 D9A, SSL-13, SSL-13 D9A, SSL-11, SSL-11 E5A D9A, SSL-9 and SSL-9 E5A D9A.

In this method, the sequence Gly-Gln-Phe-Coumarin is C-terminally coupled to the peptides. Chymotrypsin is encapsulated in the vesicles, and this enzyme cuts the peptide between phenylalanine and coumarin. If accordingly labeled peptides penetrate into the vesicles, Chymotrypsin acts and cleaves the Phe-Coumarin bond which sets the coumarin free and causes a bathochromic shift of the fluorescence maximum of about 50 nm. which can be determined fluorimetrically.

Large unilamellar vesicles consisting of POPC / POPG 25:75 (mol:mol) were used in a concentration of 100 µM in buffer without or with 100 µM ZnSO₄. The dermcidin-derived peptides were added in a concentration of 2 µM and the time profile of the translocation over a time period of 5 min was determined.

Figure 4A illustrates the results of the translocation of DCD-1L (bold line) and SSL-25 (thin line). The presence of Zn²⁺ ions accelerated or increased the uptake of the peptides into the vesicles. The dotted lines show the time course with Zn²⁺ and the solid lines without Zn²⁺.

In the case of SSL-25 already after 150 to 200 seconds the entire peptide has penetrated into the vesicles. In the case of DCD-1L after 300 seconds approximately 70 % (without Zn²⁺ ions) or 80 % (with Zn²⁺ ions), respectively, have penetrated into the vesicles.

An acceleration of penetration in the presence of other divalent ions, such as Ca⁺⁺, Mg⁺⁺, Co⁺⁺ or Ni⁺⁺, was not observed, and therefore, the enhancement of penetration by Zn⁺⁺ ions seems to be specific.

Furthermore, in Figure 4B the extent of translocation of SSL-25 (without Zn²⁺ ions) and several analogues as well as of several further dermcidin-derived peptides according to the present invention after 10 min is shown. The left group depicts the values of SSL-25 and its analogues with enhanced positive net charge and the right group represents the values of the C-terminal cleaved analogues ranging from SSL-23 to SSL-9. All analogues are able to translocate into vesicles including their charge mutants for example SSL-9 E5A D9A with exception of SSL-11 and SSL-9 which show a reduced translocation. Furthermore, Figure 4B clearly demonstrates that the charge mutants of all analogues from SSL-19, SSL-17, SSL-15, SSL-13, SSL-11 and SSL-9 show enhanced translocation with respect to their parent peptide.

These results demonstrate that the DDPs of the present invention, as shown on the exemplary peptides DCD-1L, SSL-25, and its C-terminal cleaved analogues in particular SSL15 and SSL13 are able to penetrate lipid membranes very efficiently and all charge mutants particularly with D9A mutation show a significantly enhanced translocation.

### Example 5 Translocation of DCD-1L and SSL-25 into BY2 Tobacco Cells

Four-day-old tobacco cells of the cell line BY2 (*Nicotiana tabacum* L. cv. Bright Yellow 2), which were in the stationary phase, were cultivated in MS medium without (-Zn) or with ZnSO₄ (+Zn, 30 µM) at 26 °C in the dark.

After incubation with 1 µM DCD-1L or SSL-25 over 2 h, 6 h or 20 h the cells were washed with medium solution and immediately examined by fluorescence microscopy. The focus level was positioned in the center of the cells and one optical section image was taken for each cell.

The degree of translocation was determined using the relative gray scale value of the optical sections, which was determined by the fluorescence signal detected in the cells from the peptides N-terminally labeled with rhodamine B. In the case of SSL-25 496 cells without and 496 cells with Zn²⁺ influence were examined. In the case of DCD-1L 501 cells without and 489 cells with Zn²⁺ influence were examined. The results are shown in Figure 5. Therein, the error bars indicate the standard deviation, which is based on the mean value of the relative gray scale value from three independent experimental series (biological replicas).

The results demonstrate, that the DDPs of the present invention, as shown on the exemplary peptides DCD-1L and SSL-25, are able to penetrate into the cells. The presence of Zn²⁺ ions accelerates the translocation significantly. Further, both example DDPs reach the relative maximum of their translocation in the presence of Zn²⁺ ions after 6 h incubation time.

### Example 6 Translocation of DCD-1L and SSL-25 into HeLa Tumor Cells

Equal numbers of HeLa tumor cells were introduced into the cavities of microtiter plates and left to stand in culture medium with FCS overnight. On the next day, the cells were washed with culture medium without FCS and preheated peptide solutions were added so that the final concentration of the dermcidin-derived peptides DCD-1L and SSL-25 were each 100 µM and that of Nona-arginine (R9) (as a reference CPP) was 2 µM.

Incubation with the peptides was performed in the absence of FCS. All peptides were N-terminally labeled with carboxyfluorescein. Further, propidium iodide (2.5 µg / ml) was added to the cells as a marker for viability and trypan blue (0.4%) was added, in order to quench the fluorescence of potentially adsorbed peptides on the cell surface.

The uptake of the peptides into the cells was determined with flow cytometry. The degree of translocation was determined using the median values of fluorescence of the detected cells as obtained from the histograms (Figure 6A), which were related to the respective values obtained with R9 (DCD-1L - Zn, SSL-25 - Zn, Figure 6B).

As can be seen from Figures 6A and 6B, DCD-1L and SSL-25 (in each case 100 µM) and R9 (2 µM) were taken up by the cells already after 2 h with an efficiency of 22 % (DCD-1L) and 13% (SSL-25) relative to R9.

The presence of Zn²⁺ ions (+Zn) did not have a significant effect on translocation (Figure 6C and 6D).

These results further support, that the DDPs of the present invention, as shown on the exemplary peptides DCD-1L and SSL-25, are able to penetrate into different kind of cells.

### Example 7 Translocation of SSL-25 into HaCaT Keratinocytes

To investigate the uptake of the DDPs of the present invention into skin cells, human keratinocytes (cell line HaCaT; [P. Boukamp, R.T. Petrussevska, D. Breitkreutz, J. Hornung, A. Markham, N.E. Fusenig, Normal Keratinization in a Spontaneously immortalized Aneuploid Human Keratinocyte Cell-Line, J Cell Biol, (1988) 106, 761-771] were used. Said cells were seeded in cell culture vessels having 8 chambers for microscopy (50,000 - 70,000 cells per chamber) and left to stand in standard culture medium overnight to allow adherence.

On the following day the medium was discarded and the cells washed with phosphate buffer. Subsequently, the cells were covered with sucrose buffer (300 mM of sucrose, 10 mM HEPES, pH 7.5) containing SSL-25 N-terminally labeled with rhodamine B at a concentration of 2 µM and optionally 100 µM ZnSO₄. The peptide was allowed to react for 15 minutes in the dark. Then the buffer was discarded and the cells were fixed with 4% paraformaldehyde, followed by washing three times with phosphate buffer. For the permeabilization of the cell membranes, the cells were incubated with 0.1% Triton^{™} X-100 in phosphate buffer for 5 min, then washed three times with phosphate buffer and incubated for 30 minutes with 1% BSA in PBS at room temperature to block the nonspecific binding sites. Subsequently, the dye solutions were added to the samples (DAPI for staining the cell nuclei and Alexa Fluor^{®} 488 phalloidin for staining the actin filaments). After additional washing with phosphate buffer for five times the cells were ready for examination with fluorescence microscopy.

943 single cells with Zn²⁺ incubation (+Zn), 1085 single cells without Zn²⁺ incubation (-Zn) as well as 23085 cells in a confluent cell layer with Zn²⁺ incubation and 21885 cells in a confluent cell layer without Zn²⁺ incubation were visually analyzed. The results are shown in Figure 7A and 7B. Therein, the error bars correspond to the standard deviation of the counted cells. The evaluation was carried out in three independent experiments.

It can be clearly seen that SSL-25 translocates into the keratinocytes, wherein there seems to be some preference for an association with the cell nuclei.

Further, taking into account the relatively short incubation time of only 15 min, the uptake into the cells must be considered as very efficient, and appears to occur preferably on a cell layer (25% vs. 8%).

### Example 8 Uptake of SSL-25 and its short analogues into HaCaT-Keratinocytes, and cytotoxicity

To investigate the uptake of shorter analogues of SSL25 and their charge mutants, the cellular translocation of SSL-13, SSL-13 D9A, SSL-11, SSL-9, SSL-9 E5A D9K, SSL-9 E5K D9A, and SSL-9 E5K D9K was evaluated in comparison to SSL-25 in HaCaT keratinocyte cell line. Cells (20,000 cells per well) were seeded in 24-well plates and allowed to grow in standard growth medium containing Fetal Bovine Serum (FBS) overnight to attain a confluency of 70%. On the following day, the medium was discarded and cells were washed with phosphate buffered saline. Subsequently the peptide was added at a final concentration of 25µM in culture medium without FBS. The cells were incubated with the peptides labeled with carboxyfluorescein at the N-terminus at 37°C, 5% CO₂ for 1 h in the dark. Following incubation of the cells with the peptides, the cells were washed three times with phosphate buffered saline. Subsequently, the cells were trypsinized using 0.25% Trypsin-EDTA for 10 min at 37°C, 5% CO₂ to allow the protease dependent degradation of adsorbed surface bound peptides and to allow the detachment of cells from the 24-well plate for further analysis.

The cellular uptake of peptides was assessed by flow cytometry. 5000 events per sample were recorded and analyzed in the flow cytometer. A mean of six measurements was plotted with standard deviations being represented by error bars. These results are shown in Figure 8A, the peptides SSL-13 D9A (81.5%) and SSL-9 E5K D9A (81.7%) show highest cellular translocation followed by SSL-11 (70%), SSL- 9 (50%) and SSL- 13 (48%). These peptides translocate in HaCaT cells more efficiently as compared to SSL-25 (31%), SSL-9 E5A D9K (28%) and SSL-9 E5K D9K (22%).

Further, to assess the toxicity exhibited by the shorter peptide analogues and their charge mutants on HaCaT keratinocytes, MTT assay was performed. Said cells (7000 cells per well) were seeded in 96-well plate and were allowed to grow overnight in standard growth medium containing FBS. On the next day, the medium was discarded and the cells were washed thrice with phosphate buffered saline and incubated with the peptides at 10µM, 25µM, 50µM and 100µM in culture medium without FBS for 24 h at 37°C, 5% CO₂. On the following day the cells were washed again with phosphate buffered saline. MTT reagent was added to cells at a final concentration of 1mg/ml in phosphate buffered saline and incubated for 2-4 h at 37°C, 5% CO₂. Addition of MTT to the said cells yielded purple formazan crystals that were dissolved by addition of DMSO and the absorbance was measured at 570 nm. The results are shown in Figure 8B, the viability of cells remained close to 100% in presence of all peptides except SSL-9 E5K D9A and SSL-9 E5K D9K at 10µM. However, the viability of the cells was reduced to 70% as the concentration of the peptides (SSL-13, SSL-13 D9A, SSL-11, SSL-9, SSL-9 E5A D9K, SSL-9 E5K D9A, and SSL-9 E5K D9K) was increased to 100µM. It can be seen from Figure 8B that high cell viability (70%) is still maintained in all samples. These results further support that shorter DDPs show efficient cell penetration as their charge mutants in HaCaT cells and these DDPs do not exhibit any significant toxicity towards cells at even at higher concentrations (100µM) for an extended period (24 h) of incubation.

These results further support, that the DDPs of the present invention, as shown on the peptides DCD-1L, SSL-25, shorter mutants of SSL-25 and charge mutants thereof, exhibit efficient cell-penetrating properties in a broad variety of cells.

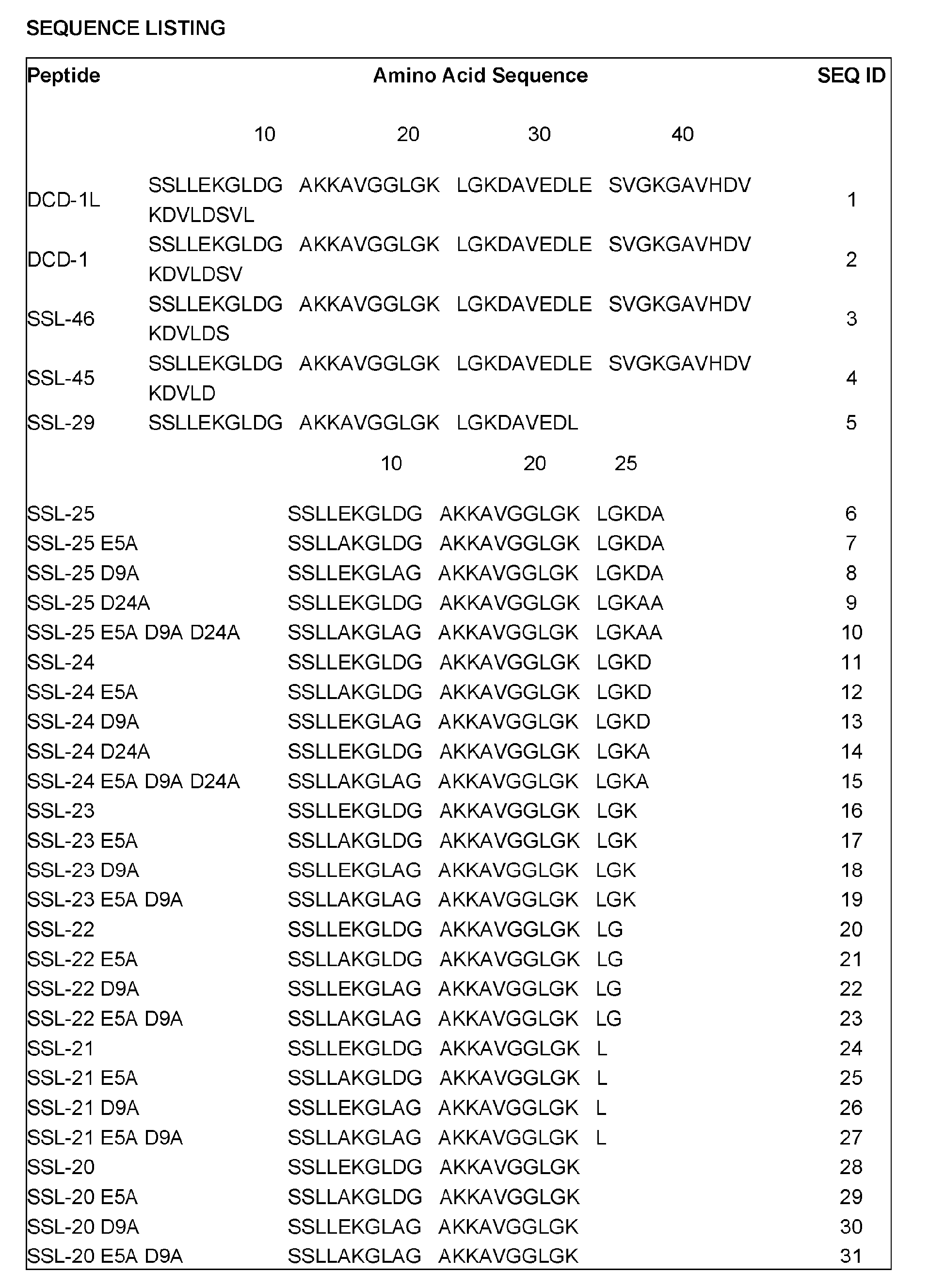

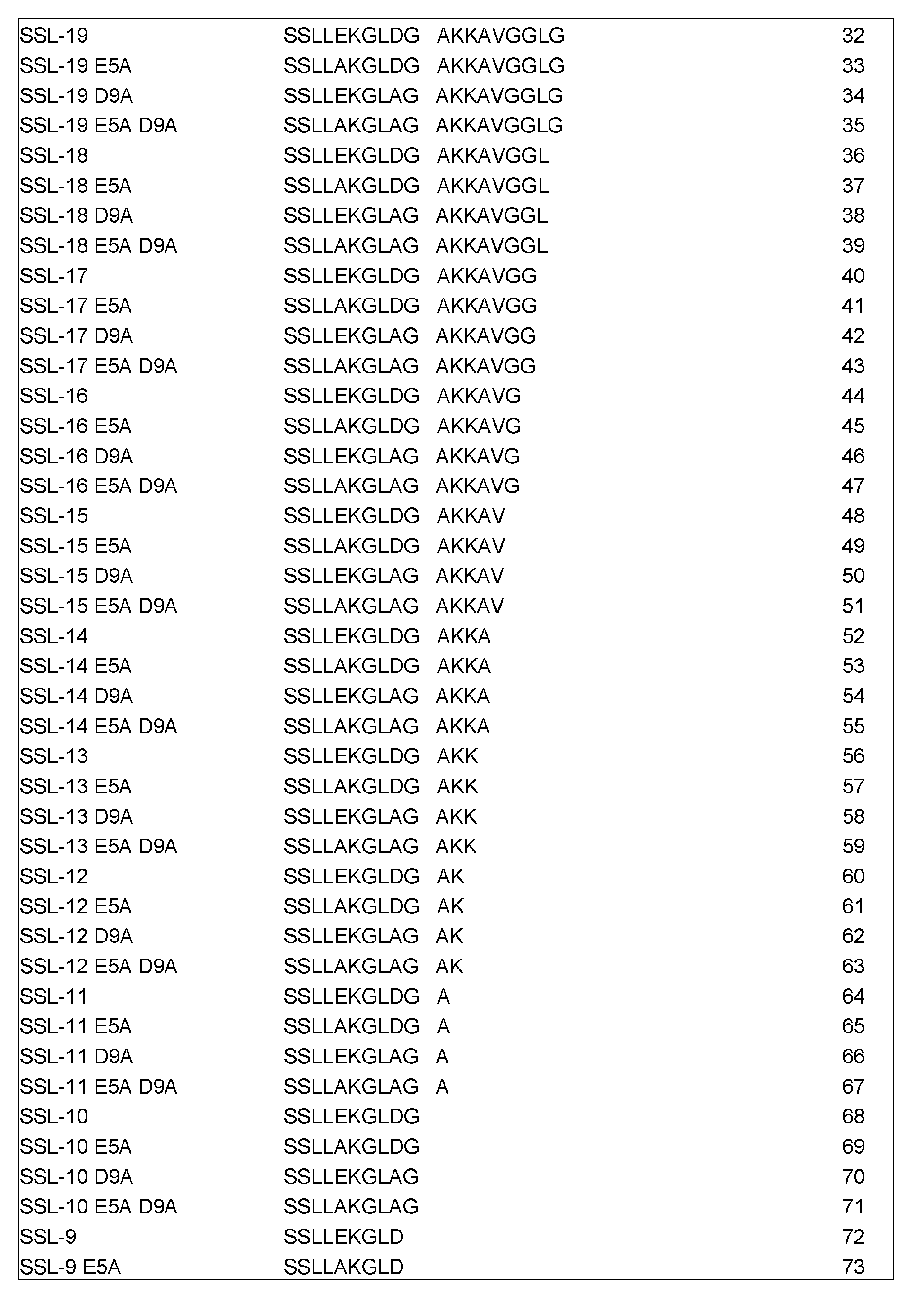

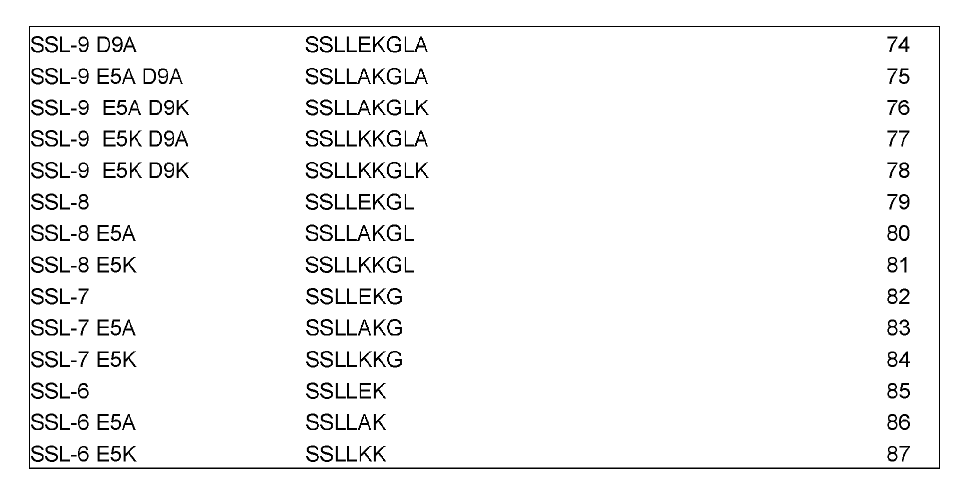

### Amino Acid Sequence SEQ ID 88 (Dermcidin)

## Claims

1. A peptide, which derives from the human dermcidin amino acid sequence (DDP), according to the following consecutive sequence of formula (I) or (II)
S-S-L-L-X1-K-G-L-X2-X3-X4-X5-X6-X7 (I)
or
S - S - L - L - X1 - K - Z1 (II)
retro-, inverso- and retroinverso-form or pharmaceutically acceptable salts or solvates thereof,
wherein
X1 is any L- or D-amino acid;
X2 is any L- or D-amino acid or is absent;
X3 is any L- or D-amino acid or is absent;
X4 is absent or an amino acid sequence comprising from 1 to 10 amino acids of the consecutive amino acid sequence A-Y1-Y2-A-V-G-G-L-G-Y3, wherein Y1, Y2 and Y3 are any L- or D-amino acid;
X5 is absent or an amino acid sequence comprising from 1 to 10 amino acids of the consecutive amino acid sequence L-G-Y4-Y5-A-V-E-D-L-E, wherein Y4 and Y5 are any L- or D-amino acid;
X6 is absent or an amino acid sequence comprising from 1 to 10 amino acids of the consecutive amino acid sequence S-V-G-K-G-A-V-H-D-V;
X7 is absent or an amino acid sequence comprising from 1 to 8 amino acids of the consecutive amino acid sequence K-D-V-L-D-S-V-L;
wherein in the case that one of X2 to X6 is absent the subsequent amino acids X3 to X7 are also absent;
Z1 is G or g or is absent;
wherein capital letters represent L-amino acids and small letters represent D-amino acids and wherein one or more L-amino acids in the formula (I) or (II) may be replaced by its corresponding D-form;
for the use as a cell-penetrating agent as a medicament, in therapy and *in vivo* diagnostics.

2. The dermcidin-derived peptide (DDP) for the use according to claim 1, wherein in the consecutive sequence according to formula (I) or (II)
X1 is E, A or K or e, a or k;
and/or in the formula (I)
X2 is D, A or K or d, a or k or is absent; and/or
X3 is G or g or is absent;
and/or
Y1, Y2, Y3, Y4 and Y5 are independently selected from the group consisting of E, A, K, D, e, a, k and d.

3. The dermcidin-derived peptide (DDP) for the use according to claim 1 or 2, wherein the dermcidin-derived peptide (DDP) is selected from the group consisting of peptides according to SEQ ID No. 1 to SEQ ID No. 87, and retro-, inverso- and retroinverso-forms thereof.

4. Use of a peptide, which derives from the human dermcidin amino acid sequence (DDP), according to the following consecutive sequence of formula (I) or (II)
S-S-L-L-X1-K-G-L-X2-X3-X4-X5-X6-X7 (I)
or
S - S - L - L - X1 - K - Z1 (II)
retro-, inverso- and retroinverso-form or pharmaceutically acceptable salts or solvates thereof,
wherein
X1 is any L- or D-amino acid;
X2 is any L- or D-amino acid or is absent;
X3 is any L- or D-amino acid or is absent;
X4 is absent or an amino acid sequence comprising from 1 to 10 amino acids of the consecutive amino acid sequence A-Y1-Y2-A-V-G-G-L-G-Y3, wherein Y1, Y2 and Y3 are any L- or D-amino acid;
X5 is absent or an amino acid sequence comprising from 1 to 10 amino acids of the consecutive amino acid sequence L-G-Y4-Y5-A-V-E-D-L-E, wherein Y4 and Y5 are any L- or D-amino acid;
X6 is absent or an amino acid sequence comprising from 1 to 10 amino acids of the consecutive amino acid sequence S-V-G-K-G-A-V-H-D-V;
X7 is absent or an amino acid sequence comprising from 1 to 8 amino acids of the consecutive amino acid sequence K-D-V-L-D-S-V-L;
wherein in the case that one of X2 to X6 is absent the subsequent amino acids X3 to X7 are also absent;
Z1 is G or g or is absent;
wherein capital letters represent L-amino acids and small letters represent D-amino acids and wherein one or more L-amino acids in the formula (I) or (II) may be replaced by its corresponding D-form;
as a cell-penetrating agent *in vitro,* as a cosmetic agent, as an *in vitro* biotechnological marker, as an *in vitro* diagnostic agent or as a carrier for the transport of a cargo molecule into cells.

5. Use of the dermcidin-derived peptide (DDP) according to claim 4, wherein in the consecutive sequence according to formula (I) or (II)
X1 is E, A or K or e, a or k;
and/or in the formula (I)
X2 is D, A or K or d, a or k or is absent; and/or
X3 is G or g or is absent;
and/or
Y1, Y2, Y3, Y4 and Y5 are independently selected from the group consisting of E, A, K, D, e, a, k and d.

6. Use of the dermcidin-derived peptide (DDP) according to claim 4 or 5, wherein the dermcidin-derived peptide (DDP) is selected from the group consisting of peptides according to SEQ ID No. 1 to SEQ ID No. 87, and retro-, inverso- and retroinverso-forms thereof.

7. A peptide, which derives from the human dermcidin amino acid sequence (DDP), according to the following consecutive sequence of formula (I) or (II)
S-S-L-L-X1-K-G-L-X2-X3-X4-X5-X6-X7 (I)
or
S - S - L - L - X1 - K - Z1 (II)
retro-, inverso- and retroinverso-form or pharmaceutically acceptable salts or solvates thereof,
wherein
X1 is any L- or D-amino acid;
X2 is any L- or D-amino acid or is absent;
X3 is any L- or D-amino acid or is absent;
X4 is absent or an amino acid sequence comprising from 1 to 10 amino acids of the consecutive amino acid sequence A-Y1-Y2-A-V-G-G-L-G-Y3, wherein Y1, Y2 and Y3 are any L- or D-amino acid;
X5 is absent or an amino acid sequence comprising from 1 to 10 amino acids of the consecutive amino acid sequence L-G-Y4-Y5-A-V-E-D-L-E, wherein Y4 and Y5 are any L- or D-amino acid;
X6 is absent or an amino acid sequence comprising from 1 to 10 amino acids of the consecutive amino acid sequence S-V-G-K-G-A-V-H-D-V;
X7 is absent or an amino acid sequence comprising from 1 to 8 amino acids of the consecutive amino acid sequence K-D-V-L-D-S-V-L;
wherein in the case that one of X2 to X6 is absent the subsequent amino acids X3 to X7 are also absent;
Z1 is G or g or is absent;
wherein capital letters represent L-amino acids and small letters represent D-amino acids and wherein one or more L-amino acids in the formula (I) or (II) may be replaced by its corresponding D-form,
which is coupled to a cargo molecule for the transport into cells, which cargo molecule is preferably selected from the group consisting of bioactive molecules, polymers, such as in particular hydrophilic polymers, nanoparticles, peptides, polypeptides, proteins, antibodies, recombinant proteins, small molecular substances, nucleic acids, mononucleotides, oligonucleotides, polynucleotides, antisense-molecules, double stranded or single stranded DNA, RNA, antisense-RNA molecules, siRNA molecule, molecular active agents, pharmaceutically active agents or drugs, cosmetically active agents, biotechnologically active agents, diagnostic agents, dyes, such as in particular fluorescence and NIR-dyes, and contrast agents or transfection reagents attached covalently or complexed non-covalently; or
which is coupled to a target cell recognition unit, which is a peptide or amino acid sequence specific to the target cell type and to which the DDP can be coupled, such as a tumor cell specific marker, to promote targeted delivery into cells; or
which is coupled to a cell-penetrating peptide (CPP), forming a CPP-coupled DDP.

8. The coupled dermcidin-derived peptides (DDP) according to claim 7 for the use as a cell-penetrating agent, as a medicament, in therapy, or *in vivo* diagnostics.

9. Use of the coupled dermcidin-derived peptides (DDP) according to claim 7 as a cell-penetrating agent, which is a cosmetic agent, an *in vitro* biotechnological marker, an *in vitro* diagnostic agent or a carrier for the *in vitro* transport of a cargo molecule into cells.

10. A composition comprising
i) at least one dermcidin-derived peptide (DDP) as defined in any one of the claims 1 to 3 and
ii) at least one cargo molecule as defined in claim 7; and/or
iii) at least one further pharmaceutically active agent;
iv) and optionally at least one solvent, auxiliary and/or excipient; and
v) optionally further comprising Zn²⁺ ions or a Zn²⁺ source,
for the use as a cell-penetrating agent as a medicament, in therapy and *in vivo* diagnostics.

11. Use of a composition comprising
i) at least one dermcidin-derived peptide (DDP) as defined in any one of the claims 1 to 3 and
ii) at least one cargo molecule as defined in claim 7; and/or
iii) at least one cosmetically active agent or biotechnological marker;
iv) and optionally at least one solvent, auxiliary and/or excipient; and
v) optionally further comprising Zn²⁺ ions or a Zn²⁺ source,
as a cell-penetrating agent *in vitro,* such as a cell-penetrating cosmetic agent or a cell-penetrating *in vitro* biotechnological marker.

12. A Kit-of-parts combination comprising
i) at least one dermcidin-derived peptide (DDP) as defined in any one of the preceding claims 1 to 3 and
ii) at least one cargo molecule as defined in claim 7; and/or
iii) Zn²⁺ ions or a Zn²⁺ source; and/or
iv) additional active agents, auxiliary substances, solvents and/or excipients, which are suitable for the intended use of said kit-of-parts combination
with the individual compounds being present in a separated spatial arrangement, for the use as a cell-penetrating agent as a medicament, in therapy and *in vivo* diagnostics.

13. Use of a Kit-of-parts combination comprising
i) at least one dermcidin-derived peptide (DDP) as defined in any one of the preceding claims 1 to 3 and
ii) at least one cargo molecule as defined in claim 7; and/or
iii) Zn²⁺ ions or a Zn²⁺ source; and/or
iv) additional active agents, auxiliary substances, solvents and/or excipients, which are suitable for the intended use of said kit-of-parts combination
as a cell-penetrating agent *in vitro,* such as a cell-penetrating cosmetic agent or a cell-penetrating *in vitro* biotechnological marker.

## Patentansprüche

1. Ein Peptid, das sich von der menschlichen Dermcidin-Aminosäuresequenz (DDP) ableitet, gemäß der folgenden konsekutiven Sequenz der Formel (I) oder (II)
**S-S-L-L-X1-K-G-L-X2-X3-X4-X5-X6-X7** (I)
oder
**S - S - L - L - X1 - K - Z1** (II)
Retro-, Invers- und Retroinversoform oder pharmazeutisch akzeptable Salze oder Solvate davon,
worin
X1 eine beliebige L- oder D-Aminosäure ist;
X2 eine beliebige L- oder D-Aminosäure ist oder nicht vorhanden ist;
X3 eine beliebige L- oder D-Aminosäure ist oder nicht vorhanden ist;
X4 nicht vorhanden ist oder eine Aminosäuresequenz, umfassend 1 bis 10 Aminosäuren der konsekutiven Aminosäuresequenz A-Y1-Y2-A-V-G-G-L-G-Y3, worin Y1, Y2 und Y3 eine beliebige L- oder D-Aminosäure sind;
X5 nicht vorhanden ist oder eine Aminosäuresequenz, umfassend 1 bis 10 Aminosäuren der konsekutiven Aminosäuresequenz L-G-Y4-Y5-A-V-E-D-L-E, worin Y4 und Y5 eine beliebige L- oder D-Aminosäure sind;
X6 nicht vorhanden ist oder eine Aminosäuresequenz, umfassend 1 bis 10 Aminosäuren der konsekutiven Aminosäuresequenz S-V-G-K-G-A-V-H-D-V;
X7 nicht vorhanden ist oder eine Aminosäuresequenz ist, umfassend 1 bis 8 Aminosäuren der konsekutiven Aminosäuresequenz K-D-V-L-D-S-V-L;
wobei für den Fall, dass eine der Aminosäuren X2 bis X6 nicht vorhanden ist, die nachfolgenden Aminosäuren X3 bis X7 ebenfalls nicht vorhanden sind;
Z1 G oder g ist oder nicht vorhanden ist;
wobei Großbuchstaben für L-Aminosäuren und Kleinbuchstaben für D-Aminosäuren stehen und wobei eine oder mehrere L-Aminosäuren in der Formel (I) oder (II) durch ihre entsprechende D-Form ersetzt sein können;
zur Verwendung als zelleindringendes Mittel als Medikament, in der Therapie und *in vivo-*Diagnostik.

2. Das von Dermcidin abgeleitete Peptid (DDP) zur Verwendung nach Anspruch 1, **dadurch gekennzeichnet, dass** in der konsekutiven Sequenz gemäß Formel (I) oder (II)
X1 E, A oder K oder e, a oder k ist;
und/oder in der Formel (I)
X2 D, A oder K oder d, a oder k ist oder nicht vorhanden ist; und/oder
X3 G oder g ist oder nicht vorhanden ist;
und/oder
Y1, Y2, Y3, Y4 und Y5 unabhängig voneinander ausgewählt sind aus der Gruppe
bestehend aus
E, A, K, D, e, a, k und d.

3. Das von Dermcidin abgeleitete Peptid (DDP) zur Verwendung gemäß Anspruch 1 oder 2, wobei das von Dermcidin abgeleitete Peptid (DDP) ausgewählt ist aus der Gruppe bestehend aus Peptiden gemäß SEQ ID Nr. 1 bis SEQ ID Nr. 87 und Retro-, Invers- und Retroinversoformen davon.

4. Verwendung eines Peptids, das sich von der menschlichen Dermcidin-Aminosäuresequenz (DDP) ableitet, gemäß der folgenden konsekutiven Sequenz der Formel (I) oder (II)
**S - S - L - L - X1 - K - G - L - X2 - X3 - X4 - X5 - X6 - X7** (I)
oder
**S - S - L - L - X1 - K - Z1** (II)
Retro-, Invers- und Retroinversoform oder pharmazeutisch akzeptable Salze oder Solvate davon,
worin
X1 eine beliebige L- oder D-Aminosäure ist;
X2 eine beliebige L- oder D-Aminosäure ist oder nicht vorhanden ist;
X3 eine beliebige L- oder D-Aminosäure ist oder nicht vorhanden ist;
X4 nicht vorhanden ist oder eine Aminosäuresequenz, umfassend 1 bis 10 Aminosäuren der konsekutiven Aminosäuresequenz A-Y1-Y2-A-V-G-G-L-G-Y3, worin Y1, Y2 und Y3 eine beliebige L- oder D-Aminosäure sind;
X5 nicht vorhanden ist oder eine Aminosäuresequenz, umfassend 1 bis 10 Aminosäuren der konsekutiven Aminosäuresequenz L-G-Y4-Y5-A-V-E-D-L-E, worin Y4 und Y5 eine beliebige L- oder D-Aminosäure sind;
X6 nicht vorhanden ist oder eine Aminosäuresequenz, umfassend 1 bis 10 Aminosäuren der konsekutiven Aminosäuresequenz S-V-G-K-G-A-V-H-D-V;
X7 nicht vorhanden ist oder eine Aminosäuresequenz ist, umfassend 1 bis 8 Aminosäuren der konsekutiven Aminosäuresequenz K-D-V-L-D-S-V-L;
wobei für den Fall, dass eine der Aminosäuren X2 bis X6 nicht vorhanden ist, die nachfolgenden Aminosäuren X3 bis X7 ebenfalls nicht vorhanden sind;
Z1 G oder g ist oder nicht vorhanden ist;
wobei Großbuchstaben für L-Aminosäuren und Kleinbuchstaben für D-Aminosäuren stehen und wobei eine oder mehrere L-Aminosäuren in der Formel (I) oder (II) durch ihre entsprechende D-Form ersetzt sein können;
als zelleindringendes *in vitro*-Mittel, als kosmetisches Mittel, als biotechnologischer *in vitro*-Marker, als *in vitro*-Diagnostikum oder als Träger für den Transport eines Frachtmoleküls in die Zellen.

5. Verwendung des von Dermcidin abgeleiteten Peptids (DDP) gemäß Anspruch 4, worin in der konsekutiven Sequenz gemäß Formel (I) oder (II)
X1 E, A oder K oder d, a oder k ist;
und/oder in der Formel (I)
X2 D, A oder K oder d, a oder k ist oder nicht vorhanden ist; und/oder
X3 G oder g ist oder nicht vorhanden ist;
und/oder
Y1, Y2, Y3, Y4 und Y5 unabhängig voneinander ausgewählt sind aus der Gruppe
bestehend aus
E, A, K, D, e, a, k und d.

6. Verwendung des von Dermcidin abgeleiteten Peptids (DDP) gemäß Anspruch 4 oder 5, wobei das von Dermcidin abgeleitete Peptid (DDP) ausgewählt ist aus der Gruppe bestehend aus Peptiden gemäß SEQ ID Nr. 1 bis SEQ ID Nr. 87, und Retro-, Invers- und Retroinversoformen davon.

7. Ein Peptid, das sich von der menschlichen Dermcidin-Aminosäuresequenz (DDP) ableitet, gemäß der folgenden konsekutiven Sequenz der Formel (I) oder (II)
S-S-L-L-X1-K-G-L-X2-X3-X4-X5-X6-X7 (I)
oder
**S - S - L - L - X1 - K - Z1** (II)
Retro-, Invers- und Retroinversoform oder pharmazeutisch akzeptable Salze oder Solvate davon,
worin
X1 eine beliebige L- oder D-Aminosäure ist;
X2 eine beliebige L- oder D-Aminosäure ist oder nicht vorhanden ist;
X3 eine beliebige L- oder D-Aminosäure ist oder nicht vorhanden ist;
X4 nicht vorhanden ist oder eine Aminosäuresequenz, umfassend 1 bis 10 Aminosäuren der konsekutiven Aminosäuresequenz A-Y1-Y2-A-V-G-G-L-G-Y3, worin
Y1, Y2 und Y3 eine beliebige L- oder D-Aminosäure sind;
X5 nicht vorhanden ist oder eine Aminosäuresequenz, umfassend 1 bis 10 Aminosäuren der konsekutiven Aminosäuresequenz L-G-Y4-Y5-A-V-E-D-L-E, worin Y4 und Y5 eine beliebige L- oder D-Aminosäure sind;
X6 nicht vorhanden ist oder eine Aminosäuresequenz, umfassend 1 bis 10 Aminosäuren der konsekutiven Aminosäuresequenz S-V-G-K-G-A-V-H-D-V;
X7 nicht vorhanden ist oder eine Aminosäuresequenz ist, umfassend 1 bis 8 Aminosäuren der konsekutiven Aminosäuresequenz K-D-V-L-D-S-V-L;
wobei für den Fall, dass eine der Aminosäuren X2 bis X6 nicht vorhanden ist, die nachfolgenden Aminosäuren X3 bis X7 ebenfalls nicht vorhanden sind;
Z1 G oder g ist oder nicht vorhanden ist;
wobei Großbuchstaben für L-Aminosäuren und Kleinbuchstaben für D-Aminosäuren stehen und wobei eine oder mehrere L-Aminosäuren in der Formel (I) oder (II) durch ihre entsprechende D-Form ersetzt sein können;
die mit einem Frachtmolekül für den Transport in die Zellen gekoppelt ist, das Frachtmolekül vorzugsweise ausgewählt ist aus der Gruppe bestehend aus bioaktiven Molekülen, Polymeren, wie insbesondere hydrophilen Polymeren, Nanopartikeln, Peptiden, Polypeptiden, Proteinen, Antikörpern, rekombinanten Proteinen, niedermolekularen Substanzen, Nukleinsäuren, Mononukleotiden, Oligonukleotiden, Polynukleotiden, Antisense-Moleküle, doppel- oder einzelsträngige DNA, RNA, Antisense-RNA-Moleküle, siRNA-Moleküle, molekulare Wirkstoffe, pharmazeutische Wirkstoffe oder Arzneimittel, kosmetische Wirkstoffe, biotechnologische Wirkstoffe, Diagnostika, Farbstoffe, wie insbesondere Fluoreszenz- und NIR-Farbstoffe, und Kontrastmittel oder Transfektionsreagenzien, die kovalent oder nicht-kovalent komplexiert sind; oder
das an eine Zielzellenerkennungseinheit gekoppelt ist, bei der es sich um eine für den Typ der Zielzelle spezifische Peptid- oder Aminosäuresequenz handelt, an die das DDP gekoppelt werden kann, wie z. B. einen zellspezifischen Marker, um die gezielte Abgabe in die Zellen zu fördern; oder das an ein zelleindringendes Peptid (CPP) gekoppelt ist, wodurch ein CPP-gekoppeltes DDP gebildet wird.

8. Das gekoppelte, von Dermcidin abgeleitete Peptid (DDP) gemäß Anspruch 7 zur Verwendung als zelleindringendes Mittel, als Medikament, in der Therapie oder *in vivo-*Diagnostik.

9. Verwendung des gekoppelten, von Dermcidin abgeleiteten Peptids (DDP) gemäß Anspruch 7 als zelleindringendes Mittel, das ein kosmetisches Mittel, ein biotechnologischer *in* vitro-Marker, ein *in vitro*-Diagnostikum oder ein Träger für den *in vitro*-Transport eines Frachtmoleküls in die Zellen ist.

10. Eine Zusammensetzung, umfassend
i) mindestens ein von Dermcidin abgeleitetes Peptid (DDP), wie es in einem der Ansprüche 1 bis 3 definiert ist und
ii) mindestens ein Frachtmolekül, wie in Anspruch 7 definiert; und/oder
iii) mindestens einen weiteren pharmazeutischen Wirkstoff;
iv) und gegebenenfalls mindestens ein Lösungsmittel, Hilfs- und/oder Arzneimittel; und
v) gegebenenfalls zusätzlich umfassend Zn²⁺-Ionen oder eine Zn²⁺-Quelle,
für die Verwendung als zelleindringendes Mittel als Medikament, in der Therapie und *in vivo*-Diagnostik.

11. Verwendung einer Zusammensetzung, umfassend
i) mindestens ein von Dermcidin abgeleitetes Peptid (DDP), wie es in einem der Ansprüche 1 bis 3 definiert ist und
ii) mindestens ein Frachtmolekül, wie in Anspruch 7 definiert; und
iii) mindestens ein kosmetischer Wirkstoff oder biotechnologischer Marker;
iv) und gegebenenfalls mindestens ein Lösungsmittel, Hilfs- und/oder Arzneimittel; und
vi) gegebenenfalls zusätzlich umfassend Zn²⁺-Ionen oder eine Zn²⁺-Quelle,
als ein zelleindringendes *in vitro*-Mittel, wie ein zelleindringendes kosmetisches Mittel oder ein zelleindringender biotechnologischer *in vitro*-Marker.

12. Eine Kit-of-parts-Kombination, umfassend
i) mindestens ein von Dermcidin abgeleitetes Peptid (DDP), wie es in einem der vorhergehenden Ansprüche 1 bis 3 definiert ist, und
ii) mindestens ein Frachtmolekül, wie in Anspruch 7 definiert; und/oder
iii) Zn²⁺-Ionen oder eine Zn²⁺-Quelle; und/oder
iv) zusätzliche Wirkstoffe, Hilfsstoffe, Lösungsmittel und/oder Arzneimittel, die für die beabsichtigte Verwendung der Kit-of-Parts-Kombination geeignet sind,
wobei die einzelnen Komponenten in getrennter räumlicher Anordnung vorliegen, zur Verwendung als zelleindringendes Mittel als Medikament, in der Therapie und in der *in* vivo-Diagnostik.

13. Verwendung einer Kit-of-parts-Kombination, umfassend
i) mindestens ein von Dermcidin abgeleitetes Peptid (DDP), wie es in einem der vorhergehenden Ansprüche 1 bis 3 definiert ist und
ii) mindestens ein Frachtmolekül, wie in Anspruch 7 definiert; und/oder
iii) Zn²⁺-Ionen oder eine Zn²⁺-Quelle; und/oder
iv) zusätzliche Wirkstoffe, Hilfsstoffe, Lösungsmittel und/oder Arzneimittel, die für die beabsichtigte Verwendung der Kit-of-parts-Kombination geeignet sind
als zelleindringendes *in vitro*-Mittel, wie z.B. ein zelleindringendes kosmetisches Mittel oder ein zelleindringender biotechnologischer *in vitro*-Marker.

## Revendications

1. Peptide, qui dérive de la séquence d'acides aminés de la dermcidine humaine (DDP), selon la séquence consécutive suivante de formule (I) ou (II)
**S - S - L - L - X1 - K - G - L - X2 - X3 - X4 - X5 - X6 - X7** (I)
or
**S - S - L - L - X1 - K - Z1** (II)
les formes rétro, inverso et rétro-inverso ou les sels ou solvates pharmaceutiquement acceptables de celui-ci,
dans lequel
X1 est l'un quelconque d'un acide aminé L ou D ;
X2 est l'un quelconque d'un acide aminé L ou D ou est absent ;
X3 est l'un quelconque d'un acide aminé L ou D ou est absent ;
X4 est absent ou une séquence d'acides aminés comprenant de 1 à 10 acides aminés de la séquence consécutive d'acides aminés A-Y1-Y2-A-V-G-G-L-G-Y3, dans lequel
Y1, Y2 et Y3 sont l'un quelconque d'un acide aminé L ou D ;
X5 est absent ou une séquence d'acides aminés comprenant de 1 à 10 acides aminés de la séquence consécutive d'acides aminés L-G-Y4-Y5-A-V-E-D-L-E, dans lequel
Y4 et Y5 sont l'un quelconque d'un acide aminé L ou D ;
X6 est absent ou une séquence d'acides aminés comprenant de 1 à 10 acides aminés de la séquence consécutive d'acides aminés S-V-G-K-G-A-V-H-D-V ;
X7 est absent ou une séquence d'acides aminés comprenant de 1 à 8 acides aminés de la séquence consécutive d'acides aminés K-D-V-L-D-S-V-L ;
dans lequel dans le cas où l'un de X2 à X6 est absent, les acides aminés suivants X3 à X7 sont également absents ;
Z1 est G ou g ou est absent ;
dans lequel les lettres majuscules représentent des acides aminés L et les lettres minuscules représentent des acides aminés D et dans lequel un ou plusieurs acides aminés L dans la formule (I) ou (II) peuvent être remplacés par sa forme D correspondante ; à utiliser comme agent de pénétration cellulaire, comme médicament, dans une thérapie et dans des diagnostics in vivo.

2. Peptide dérivé de la dermcidine (DDP) pour l'utilisation selon la revendication 1, dans lequel la séquence consécutive selon la formule (I) ou (II)
X1 est E, A ou K ou e, a ou k;
et/ou dans la formule (I)
X2 est D, A ou K ou d, a ou k ou est absent ; et/ou
X3 est G ou g ou est absent ;
et/ou
Y1, Y2, Y3, Y4 et Y5 sont sélectionnés indépendamment dans le groupe consistant en E, A, K, D, e, a, k et d.

3. Peptide dérivé de la dermcidine (DDP) pour l'utilisation selon la revendication 1 ou 2, dans lequel le peptide dérivé de la dermcidine (DDP) est sélectionné dans le groupe consistant en les peptides selon la SEQ ID No. 1 à SEQ ID No. 87, et les formes rétro, inverso et rétroinverso de ceux-ci.

4. Utilisation d'un peptide, qui dérive de la séquence d'acides aminés de la dermcidine humaine (DDP), selon la séquence consécutive suivante de formule (I) ou (II)
**S - S- L - L- X1 - K - G - L - X2 - X3 - X4 - X5 - X6 - X7** (I)
or
**S - S - L - L - X1 - K - Z1** (II)
les formes rétro, inverso et rétroinverso ou les sels ou solvates pharmaceutiquement acceptables de celui-ci,
dans laquelle
X1 est l'un quelconque d'un acide aminé L ou D ;
X2 est l'un quelconque d'un acide aminé L ou D ou est absent ;
X3 est l'un quelconque d'un acide aminé ou D ou est absent ;
X4 est absent ou une séquence d'acides aminés comprenant de 1 à 10 acides aminés de la séquence consécutive d'acide aminé A-Y1-Y2-A-V-G-G-L-G-Y3, dans laquelle
Y1, Y2 et Y3 sont l'un quelconque d'un acide aminé L ou D ;
X5 est absent ou une séquence d'acides aminés comprenant de 1 à 10 acides aminés de la séquence consécutive d'acides aminés L-G-Y4-Y5-A-V-E-D-L-E, dans laquelle
Y4 et Y5 sont l'un quelconque d'un acide aminé L ou D ;
X6 est absent ou une séquence d'acides aminés comprenant de 1 à 10 acides aminés de la séquence consécutive d'acides aminés S-V-G-K-G-A-V-H-D-V ;
X7 est absent ou une séquence d'acides aminés comprenant de 1 à 8 acides aminés de la séquence consécutive d'acides aminés K-D-V-L-D-S-V-L ;
dans laquelle dans le cas où l'un de X2 à X6 est absent, les acides aminés suivants X3 à X7 sont également absents ;
Z1 est G ou g ou est absent ;
dans laquelle les lettres majuscules représentent des acides aminés L et les lettres minuscules représentent des acides aminés D et dans laquelle un ou plusieurs acides aminés L dans la formule (I) ou (II) peuvent être remplacés par sa forme D correspondante ;
comme agent de pénétration cellulaire in vitro, comme agent cosmétique, comme marqueur biotechnologique in vitro, comme agent de diagnostic in vitro ou comme support pour le transport d'une molécule cargo dans des cellules.

5. Utilisation du peptide dérivé de la dermcidine (DDP) selon la revendication 4, dans laquelle dans la séquence consécutive selon la formule (I) ou (II)
X1 est E, A ou K ou e, a ou k ;
et/ou dans la formule (I)
X2 est D, A ou K ou d, a ou k ou est absent ; et/ou
X3 est G ou g ou est absent ;
et/ou
Y1, Y2, Y3, Y4 et Y5 sont sélectionnés indépendamment dans le groupe consistant en E, A, K, D, e, a, k et d.

6. Utilisation du peptide dérivé de la dermcidine (DDP) selon la revendication 4 ou 5, dans laquelle le peptide dérivé de la dermcidine (DDP) est sélectionné dans le groupe consistant en les peptides selon la SEQ ID No. 1 à SEQ ID No. 87, et les formes rétro, inverso et rétroinverso de ceux-ci.

7. Peptide, qui dérive de la séquence d'acides aminés de la dermcidine humaine (DDP), selon la séquence consécutive suivante de formule (I) ou (II)
**S - S - L - L - X1 - K - G - L - X2 - X3 - X4 - X5 - X6 - X7** (I)
or
**S - S - L - L - X1 - K - Z1** (II)
les formes rétro, inverso et rétroinverso ou les sels ou solvates pharmaceutiquement acceptables de celui-ci, dans lequel
X1 est l'un quelconque d'un acide aminé L ou D
X2 est l'un quelconque d'un acide aminé L ou D ou est absent ;
X3 est l'un quelconque d'un acide aminé L ou D ou est absent ;
X4 est absent ou une séquence d'acides aminés comprenant de 1 à 10 acides aminés de la séquence consécutive d'acides aminés A-Y1-Y2-A-V-G-G-L-G-Y3, dans lequel
Y1, Y2 et Y3 sont l'un quelconque d'un acide aminé L ou D ;
X5 est absent ou une séquence d'acides aminés comprenant de 1 à 10 acides aminés de la séquence consécutive d'acides aminés L-G-Y4-Y5-A-V-E-D-L-E, dans lequel
Y4 et Y5 sont l'un quelconque d'un acide aminé L ou D ;
X6 est absent ou une séquence d'acides aminés comprenant de 1 à 10 acides aminés de la séquence consécutive d'acides aminés S-V-G-K-G-A-V-H-D-V ;
X7 est absent ou une séquence d'acides aminés comprenant de 1 à 8 acides aminés de la séquence consécutive d'acides aminés K-D-V-L-D-S-V-L ; dans lequel dans le cas où l'un de X2 à X6 est absent, les acides aminés suivants X3 à X7 sont également absents ;
Z1 est G ou g ou est absent ;
dans lequel les lettres majuscules représentent des acides aminés L et les lettres minuscules représentent des acides aminés D et dans lequel un ou plusieurs acides aminés L dans la formule (I) ou (II) peuvent être remplacés par sa forme D correspondante,
qui est couplé à une molécule cargo pour le transport dans des cellules, laquelle molécule cargo est de préférence sélectionnée dans le groupe consistant en les molécules bioactives, les polymères, comme en particulier les polymères hydrophiles, les nanoparticules, les peptides, les polypeptides, les protéines, les anticorps, les protéines recombinantes, les petites substances moléculaires, les acides nucléiques, les mononucléotides, les oligonucléotides, les polynucléotides, les molécules antisens, un ADN double brin ou simple brin, un ARN, les molécules d'ARN antisens, une molécule d'ARNsi, les agents actifs moléculaires, les agents ou médicaments pharmaceutiquement actifs, les agents actifs en cosmétique, les agents biotechnologiquement actifs, les agents de diagnostic, les colorants, comme en particulier les colorants de fluorescence et NIR, et les agents de contraste ou des réactifs de transfection liés de manière covalente ou complexés de manière non covalente ; ou
qui est couplé à une unité de reconnaissance de cellule cible, qui est un peptide ou une séquence d'acides aminés spécifique du type de cellule cible et à laquelle la DDP peut être couplée, comme un marqueur spécifique de cellule tumorale, pour favoriser une administration ciblée dans des cellules ;
ou qui est couplé à un peptide de pénétration cellulaire (CPP), formant une DDP couplée à un CPP.

8. Peptides dérivés de la dermcidine (DDP) couplés selon la revendication 7 à utiliser comme agent de pénétration cellulaire, comme médicament, dans une thérapie, ou dans des diagnostics in vivo.

9. Utilisation des peptides dérivés de la dermcidine (DDP) couplés selon la revendication 7 comme agent de pénétration cellulaire, qui est un agent cosmétique, un marqueur biotechnologique in vitro, un agent de diagnostic in vitro ou un support pour le transport in vitro d'une molécule cargo dans des cellules.

10. Composition comprenant
i) au moins un peptide dérivé de la dermcidine (DDP) tel que défini dans l'une quelconque des revendications 1 à 3 et
ii) au moins une molécule cargo telle que définie dans la revendication 7 ; et/ou
iii) au moins un agent pharmaceutiquement actif supplémentaire ;
iv) et facultativement au moins un solvant, auxiliaire et/ou excipient ; et
v) facultativement comprenant en outre des ions Zn2+ ou une source de Zn2+, à utiliser comme agent de pénétration cellulaire, comme médicament, dans une thérapie et dans des diagnostics in vivo.

11. Utilisation d'une composition comprenant
i) au moins un peptide dérivé de la dermcidine (DDP) tel que défini dans l'une quelconque des revendications 1 à 3 et
ii) au moins une molécule cargo telle que définie dans la revendication 7 ; et/ou
iii) au moins un agent actif en cosmétique ou marqueur biotechnologique ;
iv) et facultativement au moins un solvant, auxiliaire et/ou excipient ; et
v) facultativement comprenant en outre des ions Zn2+ ou une source de Zn2+, comme agent de pénétration cellulaire in vitro, tel qu'un agent cosmétique de pénétration cellulaire ou un marqueur biotechnologique de pénétration cellulaire in vitro.

12. Combinaison de kit de parties comprenant
i) au moins un peptide dérivé de la dermcidine (DDP) tel que défini dans l'une quelconque des revendications 1 à 3 précédentes et
ii) au moins une molécule cargo telle que définie dans la revendication 7 ; et/ou
iii) des ions Zn2+ ou une source de Zn2+ ; et/ou
iv) des agents actifs supplémentaires, des substances auxiliaires, des solvants et/ou des excipients, qui sont appropriés pour l'utilisation prévue de ladite combinaison de kit de parties
les composés individuels étant présents dans un arrangement spatial séparé, à utiliser comme agent de pénétration cellulaire, comme médicament, dans une thérapie et dans des diagnostics in vivo.

13. Utilisation d'une combinaison de kit de parties comprenant
i) au moins un peptide dérivé de la dermcidine (DDP) tel que défini dans l'une quelconque des revendications 1 à 3 précédentes et
ii) au moins une molécule cargo telle que définie dans la revendication 7 ; et/ou
iii) des ions Zn2+ ou une source de Zn2+ ; et/ou
iv) des agents actifs supplémentaires, des substances auxiliaires, des solvants et/ou des excipients, qui sont appropriés pour l'utilisation prévue de ladite combinaison de kit de parties
comme agent de pénétration cellulaire in vitro, tel qu'un agent cosmétique de pénétration cellulaire ou un marqueur biotechnologique de pénétration cellulaire in vitro.


### SEQUENCE LISTING

<110> Karlsruher Institut für Technologie
<120> CELL PENETRATING PEPTIDES OF HUMAN ORIGIN
<130> H67528EP
<150> EP17156097.2
   <151> 2017-02-14
<160> 88
<170> BiSSAP 1.3.6
<210> 1
   <211> 48
   <212> PRT
   <213> Homo sapiens
<400> 1
   Ser Ser Leu Leu Glu Lys Gly Leu Asp Gly Ala Lys Lys Ala Val Gly
   1               5                   10                  15
   Gly Leu Gly Lys Leu Gly Lys Asp Ala Val Glu Asp Leu Glu Ser Val
               20                  25                  30
   Gly Lys Gly Ala Val His Asp Val Lys Asp Val Leu Asp Ser Val Leu
           35                  40                  45
<210> 2
   <211> 47
   <212> PRT
   <213> Homo sapiens
<400> 2
   Ser Ser Leu Leu Glu Lys Gly Leu Asp Gly Ala Lys Lys Ala Val Gly
   1               5                   10                  15
   Gly Leu Gly Lys Leu Gly Lys Asp Ala Val Glu Asp Leu Glu Ser Val
               20                  25                  30
   Gly Lys Gly Ala Val His Asp Val Lys Asp Val Leu Asp Ser Val
           35                  40                  45
<210> 3
   <211> 46
   <212> PRT
   <213> Homo sapiens
<400> 3
   Ser Ser Leu Leu Glu Lys Gly Leu Asp Gly Ala Lys Lys Ala Val Gly
   1               5                   10                  15
   Gly Leu Gly Lys Leu Gly Lys Asp Ala Val Glu Asp Leu Glu Ser Val
               20                  25                  30
   Gly Lys Gly Ala Val His Asp Val Lys Asp Val Leu Asp Ser
           35                  40                  45
<210> 4
   <211> 45
   <212> PRT
   <213> Homo sapiens
<210> 5
   <211> 29
   <212> PRT
   <213> Homo sapiens
<400> 5
   Ser Ser Leu Leu Glu Lys Gly Leu Asp Gly Ala Lys Lys Ala Val Gly
   1               5                   10                  15
   Gly Leu Gly Lys Leu Gly Lys Asp Ala Val Glu Asp Leu
               20                  25
<210> 6
   <211> 25
   <212> PRT
   <213> Homo sapiens
<400> 6
   Ser Ser Leu Leu Glu Lys Gly Leu Asp Gly Ala Lys Lys Ala Val Gly
   1               5                   10                  15
   Gly Leu Gly Lys Leu Gly Lys Asp Ala
               20                  25
<210> 7
   <211> 25
   <212> PRT
   <213> Homo sapiens
<400> 7
   Ser Ser Leu Leu Ala Lys Gly Leu Asp Gly Ala Lys Lys Ala Val Gly
   1               5                   10                  15
   Gly Leu Gly Lys Leu Gly Lys Asp Ala
               20                  25
<210> 8
   <211> 25
   <212> PRT
   <213> Homo sapiens
<400> 8
   Ser Ser Leu Leu Glu Lys Gly Leu Ala Gly Ala Lys Lys Ala Val Gly
   1               5                   10                  15
   Gly Leu Gly Lys Leu Gly Lys Asp Ala
               20                  25
<210> 9
   <211> 25
   <212> PRT
   <213> Homo sapiens
<400> 9
   Ser Ser Leu Leu Glu Lys Gly Leu Asp Gly Ala Lys Lys Ala Val Gly
   1               5                   10                  15
   Gly Leu Gly Lys Leu Gly Lys Ala Ala
               20                  25
<210> 10
   <211> 25
   <212> PRT
   <213> Homo sapiens
<400> 10
   Ser Ser Leu Leu Ala Lys Gly Leu Ala Gly Ala Lys Lys Ala Val Gly
   1               5                   10                  15
   Gly Leu Gly Lys Leu Gly Lys Ala Ala
               20                  25
<210> 11
   <211> 24
   <212> PRT
   <213> Homo sapiens
<400> 11
   Ser Ser Leu Leu Glu Lys Gly Leu Asp Gly Ala Lys Lys Ala Val Gly
   1               5                   10                  15
   Gly Leu Gly Lys Leu Gly Lys Asp
               20
<210> 12
   <211> 24
   <212> PRT
   <213> Homo sapiens
<400> 12
   Ser Ser Leu Leu Ala Lys Gly Leu Asp Gly Ala Lys Lys Ala Val Gly
   1               5                   10                  15
   Gly Leu Gly Lys Leu Gly Lys Asp
               20
<210> 13
   <211> 24
   <212> PRT
   <213> Homo sapiens
<400> 13
   Ser Ser Leu Leu Glu Lys Gly Leu Ala Gly Ala Lys Lys Ala Val Gly
   1               5                   10                  15
   Gly Leu Gly Lys Leu Gly Lys Asp
               20
<210> 14
   <211> 24
   <212> PRT
   <213> Homo sapiens
<400> 14
   Ser Ser Leu Leu Glu Lys Gly Leu Asp Gly Ala Lys Lys Ala Val Gly
   1               5                   10                  15
   Gly Leu Gly Lys Leu Gly Lys Ala
               20
<210> 15
   <211> 24
   <212> PRT
   <213> Homo sapiens
<400> 15
   Ser Ser Leu Leu Ala Lys Gly Leu Ala Gly Ala Lys Lys Ala Val Gly
   1               5                   10                  15
   Gly Leu Gly Lys Leu Gly Lys Ala
               20
<210> 16
   <211> 23
   <212> PRT
   <213> Homo sapiens
<400> 16
   Ser Ser Leu Leu Glu Lys Gly Leu Asp Gly Ala Lys Lys Ala Val Gly
   1               5                   10                  15
   Gly Leu Gly Lys Leu Gly Lys
               20
<210> 17
   <211> 23
   <212> PRT
   <213> Homo sapiens
<400> 17
   Ser Ser Leu Leu Ala Lys Gly Leu Asp Gly Ala Lys Lys Ala Val Gly
   1               5                   10                  15
   Gly Leu Gly Lys Leu Gly Lys
               20
<210> 18
   <211> 23
   <212> PRT
   <213> Homo sapiens
<400> 18
   Ser Ser Leu Leu Glu Lys Gly Leu Ala Gly Ala Lys Lys Ala Val Gly
   1               5                   10                  15
   Gly Leu Gly Lys Leu Gly Lys
               20
<210> 19
   <211> 23
   <212> PRT
   <213> Homo sapiens
<400> 19
   Ser Ser Leu Leu Ala Lys Gly Leu Ala Gly Ala Lys Lys Ala Val Gly
   1               5                   10                  15
   Gly Leu Gly Lys Leu Gly Lys
               20
<210> 20
   <211> 22
   <212> PRT
   <213> Homo sapiens
<400> 20
   Ser Ser Leu Leu Glu Lys Gly Leu Asp Gly Ala Lys Lys Ala Val Gly
   1               5                   10                  15
   Gly Leu Gly Lys Leu Gly
               20
<210> 21
   <211> 22
   <212> PRT
   <213> Homo sapiens
<400> 21
   Ser Ser Leu Leu Ala Lys Gly Leu Asp Gly Ala Lys Lys Ala Val Gly
   1               5                   10                  15
   Gly Leu Gly Lys Leu Gly
               20
<210> 22
   <211> 22
   <212> PRT
   <213> Homo sapiens
<400> 22
   Ser Ser Leu Leu Glu Lys Gly Leu Ala Gly Ala Lys Lys Ala Val Gly
   1               5                   10                  15
   Gly Leu Gly Lys Leu Gly
               20
<210> 23
   <211> 22
   <212> PRT
   <213> Homo sapiens
<400> 23
   Ser Ser Leu Leu Ala Lys Gly Leu Ala Gly Ala Lys Lys Ala Val Gly
   1               5                   10                  15
   Gly Leu Gly Lys Leu Gly
               20
<210> 24
   <211> 21
   <212> PRT
   <213> Homo sapiens
<400> 24
   Ser Ser Leu Leu Glu Lys Gly Leu Asp Gly Ala Lys Lys Ala Val Gly
   1               5                   10                  15
   Gly Leu Gly Lys Leu
               20
<210> 25
   <211> 21
   <212> PRT
   <213> Homo sapiens
<400> 25
   Ser Ser Leu Leu Ala Lys Gly Leu Asp Gly Ala Lys Lys Ala Val Gly
   1               5                   10                  15
   Gly Leu Gly Lys Leu
               20
<210> 26
   <211> 21
   <212> PRT
   <213> Homo sapiens
<400> 26
   Ser Ser Leu Leu Glu Lys Gly Leu Ala Gly Ala Lys Lys Ala Val Gly
   1               5                   10                  15
   Gly Leu Gly Lys Leu
               20
<210> 27
   <211> 21
   <212> PRT
   <213> Homo sapiens
<400> 27
   Ser Ser Leu Leu Ala Lys Gly Leu Ala Gly Ala Lys Lys Ala Val Gly
   1               5                   10                  15
   Gly Leu Gly Lys Leu
               20
<210> 28
   <211> 20
   <212> PRT
   <213> Homo sapiens
<400> 28
   Ser Ser Leu Leu Glu Lys Gly Leu Asp Gly Ala Lys Lys Ala Val Gly
   1               5                   10                  15
   Gly Leu Gly Lys
               20
<210> 29
   <211> 20
   <212> PRT
   <213> Homo sapiens
<400> 29
   Ser Ser Leu Leu Ala Lys Gly Leu Asp Gly Ala Lys Lys Ala Val Gly
   1               5                   10                  15
   Gly Leu Gly Lys
               20
<210> 30
   <211> 20
   <212> PRT
   <213> Homo sapiens
<400> 30
   Ser Ser Leu Leu Glu Lys Gly Leu Ala Gly Ala Lys Lys Ala Val Gly
   1               5                   10                  15
   Gly Leu Gly Lys
               20
<210> 31
   <211> 20
   <212> PRT
   <213> Homo sapiens
<400> 31
   Ser Ser Leu Leu Ala Lys Gly Leu Ala Gly Ala Lys Lys Ala Val Gly
   1               5                   10                  15
   Gly Leu Gly Lys
               20
<210> 32
   <211> 19
   <212> PRT
   <213> Homo sapiens
<400> 32
   Ser Ser Leu Leu Glu Lys Gly Leu Asp Gly Ala Lys Lys Ala Val Gly
   1               5                   10                  15
   Gly Leu Gly
<210> 33
   <211> 19
   <212> PRT
   <213> Homo sapiens
<400> 33
   Ser Ser Leu Leu Ala Lys Gly Leu Asp Gly Ala Lys Lys Ala Val Gly
   1               5                   10                  15
   Gly Leu Gly
<210> 34
   <211> 19
   <212> PRT
   <213> Homo sapiens
<400> 34
   Ser Ser Leu Leu Glu Lys Gly Leu Ala Gly Ala Lys Lys Ala Val Gly
   1               5                   10                  15
   Gly Leu Gly
<210> 35
   <211> 19
   <212> PRT
   <213> Homo sapiens
<400> 35
   Ser Ser Leu Leu Ala Lys Gly Leu Ala Gly Ala Lys Lys Ala Val Gly
     1               5                   10                  15
   Gly Leu Gly
<210> 36
   <211> 18
   <212> PRT
   <213> Homo sapiens
<400> 36
   Ser Ser Leu Leu Glu Lys Gly Leu Asp Gly Ala Lys Lys Ala Val Gly
   1               5                   10                  15
   Gly Leu
<210> 37
   <211> 18
   <212> PRT
   <213> Homo sapiens
<400> 37
   Ser Ser Leu Leu Ala Lys Gly Leu Asp Gly Ala Lys Lys Ala Val Gly
   1               5                   10                  15
   Gly Leu
<210> 38
   <211> 18
   <212> PRT
   <213> Homo sapiens
<400> 38
   Ser Ser Leu Leu Glu Lys Gly Leu Ala Gly Ala Lys Lys Ala Val Gly
   1               5                   10                  15
   Gly Leu
<210> 39
   <211> 18
   <212> PRT
   <213> Homo sapiens
<400> 39
   Ser Ser Leu Leu Ala Lys Gly Leu Ala Gly Ala Lys Lys Ala Val Gly
   1               5                   10                  15
   Gly Leu
<210> 40
   <211> 17
   <212> PRT
   <213> Homo sapiens
<400> 40
   Ser Ser Leu Leu Glu Lys Gly Leu Asp Gly Ala Lys Lys Ala Val Gly
   1               5                   10                  15
   Gly
<210> 41
   <211> 17
   <212> PRT
   <213> Homo sapiens
<400> 41
   Ser Ser Leu Leu Ala Lys Gly Leu Asp Gly Ala Lys Lys Ala Val Gly
   1               5                   10                  15
   Gly
<210> 42
   <211> 17
   <212> PRT
   <213> Homo sapiens
<400> 42
   Ser Ser Leu Leu Glu Lys Gly Leu Ala Gly Ala Lys Lys Ala Val Gly
   1               5                   10                  15
   Gly
<210> 43
   <211> 17
   <212> PRT
   <213> Homo sapiens
<400> 43
   Ser Ser Leu Leu Ala Lys Gly Leu Ala Gly Ala Lys Lys Ala Val Gly
   1 5 10 15
   Gly
<210> 44
   <211> 16
   <212> PRT
   <213> Homo sapiens
<400> 44
   Ser Ser Leu Leu Glu Lys Gly Leu Asp Gly Ala Lys Lys Ala Val Gly
   1 5 10 15
<210> 45
   <211> 16
   <212> PRT
   <213> Homo sapiens
<210> 46
   <211> 16
   <212> **PRT**
   <213> Homo sapiens
<400> **46**
   Ser Ser Leu Leu Glu Lys Gly Leu Ala Gly Ala Lys Lys Ala Val Gly
   1 5 10 15
<210> 47
   <211> 16
   <212> **PRT**
   <213> Homo sapiens
<400> **47**
   Ser Ser Leu Leu Ala Lys Gly Leu Ala Gly Ala Lys Lys Ala Val Gly
   1 5 10 15
<210> 48
   <211> 15
   <212> **PRT**
   <213> Homo sapiens
<400> **48**
   Ser Ser Leu Leu Glu Lys Gly Leu Asp Gly Ala Lys Lys Ala Val
   1 5 10 15
<210> 49
   <211> 15
   <212> PRT
   <213> Homo sapiens
<400> 49
   Ser Ser Leu Leu Ala Lys Gly Leu Asp Gly Ala Lys Lys Ala Val
   1 5 10 15
<210> 50
   <211> 15
   <212> PRT
   <213> Homo sapiens
<400> 50
   Ser Ser Leu Leu Glu Lys Gly Leu Ala Gly Ala Lys Lys Ala Val
   1 5 10 15
<210> 51
   <211> 15
   <212> PRT
   <213> Homo sapiens
<210> 52
   <211> 14
   <212> PRT
   <213> Homo sapiens
<400> 52
   Ser Ser Leu Leu Glu Lys Gly Leu Asp Gly Ala Lys Lys Ala
   1 5 10
<210> 53
   <211> 14
   <212> PRT
   <213> Homo sapiens
<400> 53
   Ser Ser Leu Leu Ala Lys Gly Leu Asp Gly Ala Lys Lys Ala
   1 5 10
<210> 54
   <211> 14
   <212> PRT
   <213> Homo sapiens
<400> 54
   Ser Ser Leu Leu Glu Lys Gly Leu Ala Gly Ala Lys Lys Ala
   1 5 10
<210> 55
   <211> 14
   <212> PRT
   <213> Homo sapiens
<400> 55
   Ser Ser Leu Leu Ala Lys Gly Leu Ala Gly Ala Lys Lys Ala
   1 5 10
<210> 56
   <211> 13
   <212> PRT
   <213> Homo sapiens
<400> 56
   Ser Ser Leu Leu Glu Lys Gly Leu Asp Gly Ala Lys Lys
   1 5 10
<210> 57
   <211> 13
   <212> PRT
   <213> Homo sapiens
<400> 57
   Ser Ser Leu Leu Ala Lys Gly Leu Asp Gly Ala Lys Lys
   1 5 10
<210> 58
   <211> 13
   <212> PRT
   <213> Homo sapiens
<400> 58
   Ser Ser Leu Leu Glu Lys Gly Leu Ala Gly Ala Lys Lys
   1 5 10
<210> 59
   <211> 13
   <212> PRT
   <213> Homo sapiens
<400> 59
   Ser Ser Leu Leu Ala Lys Gly Leu Ala Gly Ala Lys Lys
   1 5 10
<210> 60
   <211> 12
   <212> PRT
   <213> Homo sapiens
<400> 60
   Ser Ser Leu Leu Glu Lys Gly Leu Asp Gly Ala Lys
   1 5 10
<210> 61
   <211> 12
   <212> PRT
   <213> Homo sapiens
<400> 61
   Ser Ser Leu Leu Ala Lys Gly Leu Asp Gly Ala Lys
   1 5 10
<210> 62
   <211> 12
   <212> PRT
   <213> Homo sapiens
<400> 62
   Ser Ser Leu Leu Glu Lys Gly Leu Ala Gly Ala Lys
   1 5 10
<210> 63
   <211> 12
   <212> PRT
   <213> Homo sapiens
<400> 63
   Ser Ser Leu Leu Ala Lys Gly Leu Ala Gly Ala Lys
   1 5 10
<210> 64
   <211> 11
   <212> PRT
   <213> Homo sapiens
<400> 64
   Ser Ser Leu Leu Glu Lys Gly Leu Asp Gly Ala
   1 5 10
<210> 65
   <211> 11
   <212> PRT
   <213> Homo sapiens
<400> 65
   Ser Ser Leu Leu Ala Lys Gly Leu Asp Gly Ala
   1 5 10
<210> 66
   <211> 11
   <212> PRT
   <213> Homo sapiens
<400> 66
   Ser Ser Leu Leu Glu Lys Gly Leu Ala Gly Ala
   1 5 10
<210> 67
   <211> 11
   <212> PRT
   <213> Homo sapiens
<400> 67
   Ser Ser Leu Leu Ala Lys Gly Leu Ala Gly Ala
   1 5 10
<210> 68
   <211> 10
   <212> PRT
   <213> Homo sapiens
<400> 68
   Ser Ser Leu Leu Glu Lys Gly Leu Asp Gly
   1 5 10
<210> 69
   <211> 10
   <212> PRT
   <213> Homo sapiens
<400> 69
   Ser Ser Leu Leu Ala Lys Gly Leu Asp Gly
   1 5 10
<210> 70
   <211> 10
   <212> PRT
   <213> Homo sapiens
<400> 70
   Ser Ser Leu Leu Glu Lys Gly Leu Ala Gly
   1 5 10
<210> 71
   <211> 10
   <212> PRT
   <213> Homo sapiens
<400> 71
   Ser Ser Leu Leu Ala Lys Gly Leu Ala Gly
   1 5 10
<210> 72
   <211> 9
   <212> PRT
   <213> Homo sapiens
<400> 72
   Ser Ser Leu Leu Glu Lys Gly Leu Asp
   1 5
<210> 73
   <211> 9
   <212> PRT
   <213> Homo sapiens
<400> 73
   Ser Ser Leu Leu Ala Lys Gly Leu Asp
   1 5
<210> 74
   <211> 9
   <212> PRT
   <213> Homo sapiens
<400> 74
   Ser Ser Leu Leu Glu Lys Gly Leu Ala
   1 5
<210> 75
   <211> 9
   <212> PRT
   <213> Homo sapiens
<400> 75
   Ser Ser Leu Leu Ala Lys Gly Leu Ala
   1 5
<210> 76
   <211> 9
   <212> PRT
   <213> Homo sapiens
<400> 76
   Ser Ser Leu Leu Ala Lys Gly Leu Lys
   1 5
<210> 77
   <211> 9
   <212> PRT
   <213> Homo sapiens
<400> 77
   Ser Ser Leu Leu Lys Lys Gly Leu Ala
   1 5
<210> 78
   <211> 9
   <212> PRT
   <213> Homo sapiens
<400> 78
   Ser Ser Leu Leu Lys Lys Gly Leu Lys
   1 5
<210> 79
   <211> 8
   <212> PRT
   <213> Homo sapiens
<400> 79
   Ser Ser Leu Leu Glu Lys Gly Leu
   1 5
<210> 80
   <211> 8
   <212> PRT
   <213> Homo sapiens
<400> 80
   Ser Ser Leu Leu Ala Lys Gly Leu
   1 5
<210> 81
   <211> 8
   <212> PRT
   <213> Homo sapiens
<400> 81
   Ser Ser Leu Leu Lys Lys Gly Leu
   1 5
<210> 82
   <211> 7
   <212> PRT
   <213> Homo sapiens
<400> 82
   Ser Ser Leu Leu Glu Lys Gly
   1 5
<210> 83
   <211> 7
   <212> PRT
   <213> Homo sapiens
<400> 83
   Ser Ser Leu Leu Ala Lys Gly
   1 5
<210> 84
   <211> 7
   <212> PRT
   <213> Homo sapiens
<400> 84
   Ser Ser Leu Leu Lys Lys Gly
   1               5
<210> 85
   <211> 6
   <212> PRT
   <213> Homo sapiens
<400> 85
   Ser Ser Leu Leu Glu Lys
   1               5
<210> 86
   <211> 6
   <212> PRT
   <213> Homo sapiens
<400> 86
   Ser Ser Leu Leu Ala Lys
   1               5
<210> 87
   <211> 6
   <212> PRT
   <213> Homo sapiens
<400> 87
   Ser Ser Leu Leu Lys Lys
   1               5
<210> 88
   <211> 110
   <212> PRT
   <213> Homo sapiens
<400> 88
   Met Arg Phe Met Thr Leu Leu Phe Leu Thr Ala Leu Ala Gly Ala Leu
   1               5                   10                  15
   Val Cys Ala Tyr Asp Pro Glu Ala Ala Ser Ala Pro Gly Ser Gly Asn
               20                  25                  30
   Pro Cys His Glu Ala Ser Ala Ala Gln Lys Glu Asn Ala Gly Glu Asp
           35                  40                  45
   Pro Gly Leu Ala Arg Gln Ala Pro Lys Pro Arg Lys Gln Arg Ser Ser
       50                  55                  60
   Leu Leu Glu Lys Gly Leu Asp Gly Ala Lys Lys Ala Val Gly Gly Leu
   65                  70                  75                  80
   Gly Lys Leu Gly Lys Asp Ala Val Glu Asp Leu Glu Ser Val Gly Lys
                   85                  90                  95
   Gly Ala Val His Asp Val Lys Asp Val Leu Asp Ser Val Leu
               100                 105                 110